# EUROPEAN PATENT APPLICATION

(11) **EP 3 705 139 A1**
(43) Date of publication of application: **09.09.2020**
(21) Application number: 20159176.5
(22) Date of filing: 25.02.2020
(51) Int. Cl.: A61L 9/012, C11D 3/22

(54) **STRUCTURED AQUEOUS COMPOSITIONS HAVING DISPERSED LIQUID BENEFIT AGENT DROPLETS**

(30) Priority: 08.03.2019 EP 19161635; 11.03.2019 US 201962816259 P
(71) Applicant: The Procter & Gamble Company, Cincinnati, OH 45202 (US)
(72) Inventor: LYNCH, Matthew Lawrence, Cincinnati, Ohio 45202 (US); HORENZIAK, Steven Anthony, Cincinnati, Ohio 45202 (US); ILLIE, Brandon Philip, Cincinnati, Ohio 45202 (US); YEARY, Amber Joy, Cincinnati, Ohio 45202 (US); COLINA, Carla Jean, Cincinnati, Ohio 45202 (US)
(74) Representative: De Clercq & Partners

(57) **Abstract**

An aqueous composition, a sprayable composition containing the aqueous composition, and a method of making the aqueous composition are provided. The aqueous composition includes an aqueous phase and liquid benefit agent droplets discontinuously dispersed throughout the aqueous phase. The aqueous phase has a structurant system including a first polysaccharide and a second polysaccharide that is different from the first polysaccharide. The aqueous phase comprises less than 10,000 ppm surfactant. The aqueous composition exhibits a yield stress as determined by the RHEOLOGY TEST METHOD.

## Description

### FIELD

The present disclosure relates to phase-stable aqueous compositions having suspended liquid benefit agent droplets and products for spraying the phase-stable aqueous compositions.

### BACKGROUND

There is consumer demand for sprayable product containing a variety of liquid benefit agents (e.g. perfumes, conditioning oils) to treat many types of surfaces. Consumers may also have an interest in sprayable products that are also easily portable for use outside of a home or business. Consumers demand an aqueous composition that is phase-stable to prevent bulk separation of the liquid benefit agent to ensure the first and last spray is identical and to eliminate the need to shake the product before use. There may also be a need in some usages to minimize residue left on surfaces, particularly where there is potential for unsightly stains or bad feel properties. Consumers may also prefer particular spray characteristics relating to parameters such as spray droplet size, cone angle, and flow rate.

Liquid benefit agents may be water-insoluble. In order to stabilize water-insoluble liquid benefit agents in an aqueous composition, emulsification may be used. Emulsification is the process of dispersing liquid benefit agents from a bulk raw material into discrete droplets in an aqueous composition, generally using surfactants and/or emulsifiers. However, surfactants are not always successful in emulsifying liquid benefit agents or high concentrations of surfactants may be needed to emulsify some liquid benefit agents. Also, consumers increasingly demand natural-ingredient products. There is a general trend for consumers away from the use of chemicals, for safety and sustainability (et cetera), but towards natural materials. Many surfactants and/or emulsifiers may not be classified or considered as natural ingredients.

Thus, there is a need for sprayable and phase-stable aqueous compositions having suspended liquid benefit agent droplets and methods of making the phase-stable aqueous compositions. Moreover, there is a need for sprayable, phase-stable, and natural aqueous compositions having suspended liquid benefit agents and methods of making the phase-stable aqueous compositions.

### SUMMARY

An aqueous composition is provided, the aqueous composition comprising an aqueous phase and liquid benefit agent droplets homogeneously and discontinuously dispersed throughout the aqueous phase. The aqueous phase comprises a structurant system comprising a first polysaccharide and a second polysaccharide that is different from the first polysaccharide. The aqueous phase comprises less than 10,000 ppm surfactant. The aqueous composition exhibits a yield stress as determined by the RHEOLOGY TEST METHOD.

A method of formulating an aqueous composition is provided. The method comprises the steps of: dispersing a structurant system into water to form a concentrate, the structurant system comprising a first polysaccharide and a second polysaccharide that is different from the first polysaccharide; mixing a liquid benefit agent into the concentrate to form liquid benefit agent droplets dispersed throughout the concentrate; and diluting the concentrate with additional water to form the aqueous composition, wherein the concentration of the structurant system in the concentrate is greater than the concentration of the structurant system in the aqueous composition.

### BRIEF DESCRIPTION OF DRAWINGS

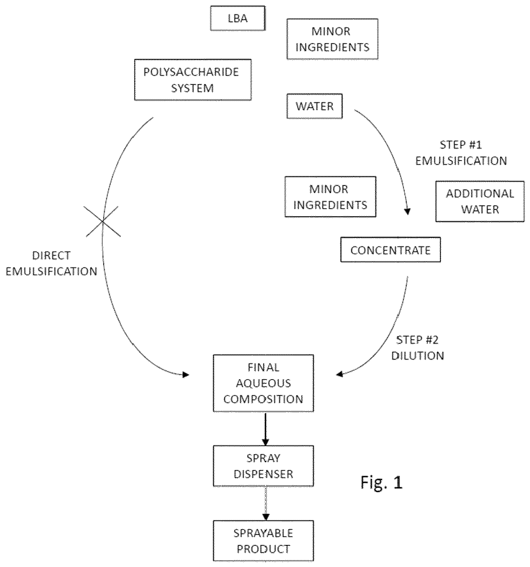

Fig. 1 is a schematic of the inventive composition, and composition and method of making. The *Direct Emulsification* route (left) combines and mixes raw materials, to create the composition. The Inventive Emulsification route (right) requires two steps: (1) Emulsification Step and 2) Dilution Step. The Emulsification Step leverages a concentrate to facilitate the emulsification of the liquid benefit agent; the Dilution Step further dilutes the concentrate to arrive at the composition.

### DETAILED DESCRIPTION

The present invention may be understood more readily by reference to the following detailed description of illustrative and preferred compositions. It is to be understood that the scope of the claims is not limited to the specific products, methods, conditions, devices, or parameters described herein, and that the terminology used herein is not intended to be limiting of the claimed invention. Also, as used in the specification, including the appended claims, the singular forms "a," "an," and "the" include the plural, and reference to a particular numerical value includes at least that particular value, unless the context clearly dictates otherwise. When a range of values is expressed, another embodiment includes from the one particular value and/or to the other particular value. Similarly, when values are expressed as approximations, by use of the antecedent basis "about," it will be understood that the particular values form another embodiment. All ranges are inclusive and combinable. All percentages and ratios used herein are by weight of the total product, and all measurements made are at 25°C, unless otherwise designated.

The following definitions may be useful in understanding the present disclosure.
"Aqueous phase" comprises water, polysaccharides and minor ingredients.
"Minor ingredients" comprise water-soluble ingredients in the aqueous phase. These include salts, organics such as glycerin and propylene diol, solvents, and/or low levels of surfactants.
"Liquid benefit agent" or "LBA" means a water-insoluble material that is liquid at 25° Celsius and that imparts a benefit to surfaces such as fabrics, hair, and skin.
"Liquid benefit agent droplet" means a droplet of liquid benefit agent that is completely surrounded by an aqueous phase. A droplet of liquid benefit agent floating on the surface of an aqueous phase is not a "liquid benefit agent droplet".
"Concentrate" includes a portion of the aqueous phase, including minor ingredients, liquid benefit agents and polysaccharides and that has more solid content than the final product composition to facilitate emulsification of the liquid benefit agent into stable droplets.
"Substantially free of' means that the component is not intentionally incorporated into the composition, but may come in to the composition as a byproduct or impurity of another component.
"Essential oil" means a concentrated hydrophobic liquid containing volatile (defined as "the tendency of a substance to vaporize") aroma compounds from plants. Essential oils are also known as volatile oils, ethereal oils, aetherolea, or simply as the oil of the plant from which they were extracted, such as oil of clove, peppermint oil, etc.
"Natural oils" are derived from renewable vegetable resources.
"Solid particle" means a particle
"ppm" means 1 mg/L per = 1 ppm.

Aqueous compositions of the present disclosure include a plurality of liquid benefit agent droplets and a structurant system to suspend the liquid benefit agent droplets. The aqueous composition may be free of, substantially free of a surfactant because the structurant system is able to suspend the liquid benefit agent droplets. The aqueous compositions may be sprayable. The aqueous compositions may be phase-stable, meaning that the liquid benefit agent droplets remain suspended in the aqueous composition for extended periods of time, eliminating the need to shake the product before use. The aqueous compositions may be used in the air or on a surface and/or to remove or reduce the amounts of malodors in the air or on a surface and/or to deliver a benefit agent into the air or onto a surface through application from a spray dispenser. A product may include a spray dispenser comprising the aqueous composition.

In order to suspend the liquid benefit agent droplets in the aqueous composition, the incorporation of the structurant system causes the aqueous composition to exhibit a yield stress. The yield stress of the aqueous composition prevents the liquid benefit agent droplets from phase-separating.

It has been found that the method of combining the structurant system with the liquid benefit agent has an effect on the phase-stability of the composition. In a typical emulsification process that utilizes a surfactant as an emulsifier, all of the raw materials are combined at one time and mixed to create the emulsified composition. However, it has been found that preparing a concentrate of the aqueous phase that includes a lower concentration of water than the final aqueous composition concentration results in a stable aqueous composition of liquid benefit agent droplets discontinuously dispersed through the aqueous phase.

Without wishing to be bound by theory, it is believed that increasing the viscosity of the concentrate to be similar or substantially similar to the viscosity of the liquid benefit agent, at least during the emulsification step, results in liquid benefit agent droplets of a small enough droplet size for the aqueous composition remain phase-stable. As the viscosities of the concentrate of aqueous phase and liquid benefit agent diverge, the resulting liquid benefit agent droplets become larger and the aqueous composition is less likely to be phase-stable. As the viscosities of the concentrate of aqueous phase and liquid benefit agent converge, the aqueous composition is phase stable and remains phase-stable even with additional dilution of water to form an aqueous composition that is more easily sprayable.

### SPRAY DISPENSER

Sprayable products may include the aqueous composition container in a spray dispenser. The spray dispenser may include a bottle for containing the aqueous composition and a spray engine.

The spray engine may be configured in various ways, such as a direct compression-type trigger sprayer, a pre-compression-type trigger sprayer, or an aerosol-type spray dispenser. One suitable spray dispenser is the TS800 Trigger Sprayer (Exxon Mobil PP1063, material classification 10003913, Manufacturer: Calmar).

Another suitable spray engine includes a continuous action sprayer, such as FLAIROSOL™ dispenser from Afa Dispensing Group. The FLAIROSOL™ dispenser includes a pre-compression spray engine and aerosol-like pressurization of the aqueous composition through the use of a pressure or buffer chamber.

A broad array of trigger sprayers or finger pump sprayers are suitable for use with the aqueous compositions of this invention. These are readily available from suppliers such as Calmar, Inc., City of Industry, Calif.; CSI (Continental Sprayers, Inc.), St. Peters, Mo.; Berry Plastics Corp., Evansville, Ind., a distributor of Guala® sprayers; or Seaquest Dispensing, 30 Cary, Ill. The preferred trigger sprayers are the blue inserted Guala® sprayer, available from Berry Plastics Corp., or the Calmar TS800-1A®, TS1300®, and TS-800-2®, available from Calmar Inc., because of the fine uniform spray 35 characteristics, spray volume, and pattern size. More preferred are sprayers with precompression features and finer spray characteristics and even distribution, such as Yoshino sprayers from Japan. Any suitable bottle or container can be used with the trigger sprayer, the preferred bottle is a 17 40 fl-oz. bottle (about 500 ml) of good ergonomics similar in shape to the Cinch® bottle. It can be made of any materials such as high-density polyethylene, polypropylene, polyvinyl chloride, polystyrene, polyethylene terephthalate, glass, or any other material that forms bottles. Preferably, it is made 45 of high density polyethylene or clear polyethylene terephthalate.

For smaller fluid ounce sizes (such as 1 to 8 ounces, for illustrative purposes only), a finger pump can be used with canister or cylindrical bottle. The preferred pump for this application is the cylindrical Euromist II® from Seaquest Dispensing. More preferred are so those with pre-compression features.

The bottle may be configured as a container having a base and sidewall wall that terminates at an opening. The bottle may include a bag-in-bag or bag-in-can container.

The bottles may be large to enclose hundreds of grams of aqueous composition as current fabric treatment sprays. Or, the bottles may be small to enclose tens of grams of aqueous composition to allow carrying in purse or pocket for on-the-go or in-between application of aqueous composition. The bottle may have a longest dimension of less than 20 cm, preferably less than 10 cm, and most preferably less than 5 cm. The bottle may contain 0.1 milliliters ("mL") to 50 mL, more preferably 1 mL to 30 mL, most preferably 1 mL to 20 mL.

If the spray dispenser is configured as an aerosol, the spray dispenser may be pressurized with a propellant. Any suitable propellant may be used.

### AQUEOUS COMPOSITION

The aqueous composition of the present invention includes an aqueous phase and a plurality of liquid benefit agent droplets discontinuously dispersed throughout the aqueous phase. The aqueous phase includes an aqueous carrier, a structurant system, and minor ingredients. The aqueous composition may be formulated by first forming a concentrate comprising at least an aqueous phase and a structurant system. The concentrate can also include minor ingredients. The liquid benefit agent is then mixed with the concentrate to form liquid benefit agent droplets that are discontinuously dispersed throughout the concentrate. The concentrate with liquid benefit agent droplets can then be diluted with additional aqueous phase while maintaining the phase stability of the aqueous composition. The additional aqueous phase can include water, structurant system, and/or minor ingredients.

### Structurant System

In particular, the structurant system may be in the form of a polysaccharide system. Preferable polysaccharides include xanthan gum, glucomannan gums, galactomannan gums, and combinations thereof. The glucomannan may be derived from a natural gum such as konjac gum. The galactomannan may be derived from naturals gums such as locust bean gum or tara gum. Polysaccharides may also include carrageenan. One or more of the polysaccharides may be modified such as by deacetylation. The xanthan gum may be acetylated.

The aqueous composition may include a polysaccharide system comprising at least two polysaccharides, such as a first polysaccharide and a second polysaccharide. The first polysaccharide may be xanthan gum. The second polysaccharide may be selected from the group consisting of glucomannan, galactomannan, and combinations thereof. The second polysaccharide may be selected from the group consisting of konjac gum, locust bean gum, tara gum, and combinations thereof.

Without wishing to be bound by theory, it is believed that the two or more polysaccharides exhibit synergistic binding, such that the binding of sites between different polysaccharides is greater than the binding between sites between the same polysaccharide to create a structurant system capable of suspending the liquid benefit agent droplets.

The first polysaccharide may comprise 10 wt. % to 80 wt. % by weight of the polysaccharide system; the second polysaccharide may comprise 20 wt. % to 90 wt. % by weight of the polysaccharide system, but greater than the composition.

The first polysaccharide may be present at a level of greater than 10 wt. % and less than 90 wt. %, alternatively about 20 wt. % to about 80 wt. %, alternatively about 40 wt. % to about 60 wt.%, by weight of the polysaccharide system.

The second polysaccharide may be present at a level of about 15 wt. % to about 85 wt. %, alternatively about 20 wt. % to about 80 wt. %, alternatively about 40 wt. % to about 60 wt.%, by weight of the polysaccharide system.

The total polysaccharide concentration present in the aqueous composition may be greater than about 0.1 wt. %, or preferably greater than about 0.2 wt. %, or preferably greater than about 0.3 wt. %, more preferably greater than 0.4 wt.%, and most preferably greater than 0.5 wt. %. Not wishing to be bound by theory, but it is believed that the greater the overall concentration of polysaccharide in the aqueous composition, the higher the viscosity, the better the emulsification of the liquid benefit agent, and the higher the yield stress to prevent coalescence and bulk separation of the liquid benefit agent.

The polysaccharide system may have a weight-average molecular weight in the range of about 10,000 Daltons to about 15,000,000 Daltons, preferably about 200,000 Daltons to about 10,000,000 Daltons, more preferably about 500,000 Daltons to about 9,000,000 Daltons, more preferably about 750,000 Daltons to about 8,000,000 Daltons, more preferably about 1,000,000 Daltons to about 7,000,000 Daltons, more preferably about 2,000,000 Daltons to about 6,000,000 Daltons, more preferably about 3,500,000 Daltons to about 6,000,000 Daltons

The polysaccharides may be characterized by the ratio of acetylation. The ratio of acetylation of one or more of the polysaccharides in the structurant system may be in the range of about 5.0 to about 0.2, preferably in the range of about 3.5 to about 0.3, preferably in the range of about 2.0 to about 0.35, preferably in the range of about 1.5 to about 0.37, preferably in the range of about 1.0 to about 0.39.

The aqueous composition may have a total protein level of less than about 100 parts per million (ppm), preferably less than 50 ppm, preferably less than 25 ppm, more preferably less than 10 ppm. It may be desirable to limit the total protein level in the aqueous composition in order to minimize discoloring of surfaces to which the aqueous composition is applied.

### Liquid Benefit Agent Droplets

The aqueous composition may comprise a plurality of liquid benefit agent droplets that are suspended in an aqueous phase. In air or surface products, liquid benefit agents may be perfumes that provide freshness, including malodor reduction and improved scent, to surfaces such as furniture, carpets and curtains. In hair products, the liquid benefit agents may be silicone oils that provide benefits such as feel, refresh, and anti-static. In fabric treatment products, the liquid benefit agents may be liquids that provide anti-wrinkle and/or freshness benefits. The liquid benefit agent may include materials selected from the group consisting of perfume raw; silicone oils, waxes such as polyethylene waxes; essential oils such as fish oils, jasmine, camphor, lavender; skin coolants such as menthol, methyl lactate; glycerin; the like; and mixtures thereof.

Suitable benefit agents can be obtained from Givaudan Corp. of Mount Olive, New Jersey, USA, International Flavors & Fragrances Corp. of South Brunswick, New Jersey, USA, or Firmenich Company of Geneva, Switzerland.

Liquid benefit agents may include material which is used to give a particular conditioning benefit to hair and/or skin. In hair compositions, suitable liquid benefit agents include those which deliver one or more benefits relating to shine, softness, comb-ability, antistatic properties, wet-handling, damage, manageability, body, and greasiness.

Suitable liquid benefit agents include conditioning agents, for example, hair conditioners, skin conditioners, or fabric conditioners, such as silicone, petrolatum, hydrocarbon oils (e.g. mineral oil), natural and synthetic waxes (e.g. micro-crystalline waxes), paraffins, ozokerite, polyethylene, polybutene, polydecene, pentahydrosqualene, vegetable oils, triglycerides, fats, and combinations thereof. Furthermore, the liquid benefit agent can be or can comprise perfume oil. Several liquid benefit agents suitable for use herein are described below.

The liquid benefit agent droplets may be sufficiently small to form a stable suspension. For example, the liquid benefit agent droplets may have a diameter of equal to or less than 300 microns (µm), or equal to or less than 200 µm, or equal to or less than 100 µm, or equal to or less than 75 µm, or equal to or less than 40 µm, or equal to or less than 30 µm, or equal to or less than 20 µm, as determined by the DROPLET SIZE TEST METHOD described below. The liquid benefit agent droplets may include one type of liquid benefit agent or a combination of two or more different types of liquid benefit agents.

The concentration of the liquid benefit agent in the concentrate and final composition should be sufficient to provide the desired benefits. Such concentration can vary with the liquid benefit agent, the performance desired, the type and concentration of other components, and other like factors. However, increased concentration of the liquid benefit agent does affect the concentration of structurant system needed to suspend the liquid benefit agent. As such, it may be desirable to limit to some degree the concentration of the liquid benefit agent in the concentrate and final aqueous composition.

The concentration of the liquid benefit agent droplets in the concentrate may be less than or equal to 20 wt.%, or less than or equal to 10 wt.%, or less than or equal to 6 wt.%, or less than or equal to 4 wt.%, or less than or equal to 2 wt.%, or less than or equal to 1 wt.%, or less than or equal to 0.2 wt.%.

The concentration of the liquid benefit agent droplets in the final aqueous composition may be less than or equal to 5 wt.%, or less than or equal to 4 wt.%, or less than or equal to 3 wt.%, or less than or equal to 2 wt.%, or less than or equal to 1 wt.%, or less than or equal to 0.4 wt.%, or less than or equal to 0.2 wt.%.

The liquid benefit agent droplets may have a viscosity in the range of 0.001 mPa·s to 5000 mPa·s, more preferably less than 2500 mPa·s, more preferably less than 1000 mPa·s, more preferably less than 500 mPa·s, more preferably less than 100 mPa·s, and more preferably less than 10 mPa·s, as determined by VISCOSITY OF LBA TEST METHOD.

### Silicones

The liquid benefit agent of the aqueous compositions of the present invention may be a water-insoluble silicone. The silicone may comprise volatile silicone, non-volatile silicone, or combinations thereof. Preferred are non-volatile silicone conditioning agents. If volatile silicones are present, it will typically be incidental to their use as a solvent or carrier for commercially available forms of non-volatile silicone material ingredients, such as silicone gums and resins. The silicone may comprise a silicone fluid conditioning agent and may also comprise other ingredients, such as a silicone resin to improve silicone fluid deposition efficiency or enhance glossiness of the surface.

Suitable silicones are selected from the group consisting of siloxanes, silicone gums, aminosilicones, terminal aminosilicones, alkyl siloxane polymers, cationic organopolysiloxanes, and mixtures thereof.

The liquid benefit agent may comprise one or more silicones including high molecular weight polyalkyl or polyaryl siloxanes and silicone gums; lower molecular weight polydimethyl siloxane fluids; and aminosilicones.

The high molecular weight polyalkyl or polyaryl siloxanes and silicone gums have a viscosity of from about 100,000 mPa·s to about 30,000,000 mPa·s at 25°C, or from about 200,000 mPa·s to about 30,000,000 mPa·s, and a molecular weight of from about 100,000 Daltons to about 1,000,000 Daltons, or from about 120,000 Daltons to about 1,000,000 Daltons.

Preferred higher molecular weight silicone compounds useful herein include polyalkyl or polyaryl siloxanes with the following structure: 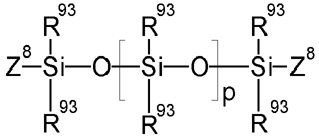 wherein R⁹³ is alkyl or aryl, and p is an integer from about 1,300 to about 15,000, more preferably from about 1,600 to about 15,000. Z⁸ represents groups which block the ends of the silicone chains. The alkyl or aryl groups substituted on the siloxane chain (R⁹³) or at the ends of the siloxane chains Z⁸ can have any structure as long as the resulting silicone remains fluid at room temperature, is dispersible, is neither irritating, toxic nor otherwise harmful when applied to the surface, is compatible with the other components of the aqueous composition, is chemically stable under normal use and storage conditions, and is capable of being deposited on and conditions the surface. Suitable Z⁸ groups include hydroxy, methyl, methoxy, ethoxy, propoxy, and aryloxy. The two R⁹³ groups on the silicon atom may represent the same group or different groups. Preferably, the two R⁹³ groups represent the same group. Suitable R⁹³ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. Commercially available silicone compounds useful herein include, for example, those available from the General Electric Company in their TSF451 series, and those available from Dow Corning in their Dow Corning SH200 series.

The silicone compounds that can be used herein can also include a silicone gum. The term "silicone gum", as used herein, means a polyorganosiloxane material having a viscosity at 25°C of greater than or equal to 1,000,000mPa·s. It is recognized that the silicone gums described herein can also have some overlap with the above-disclosed silicone compounds. This overlap is not intended as a limitation on any of these materials. The "silicone gums" will typically have a mass molecular weight in excess of about 165,000, generally between about 165,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. Commercially available silicone gums useful herein include, for example, TSE200A and CF330M available from the General Electric Company.

The lower molecular weight silicones have a viscosity of from about 1mPa·s to about 10,000mPa·s at 25°C, or from about 5mPa·s to about 5,000mPa·s,and a molecular weight of from about 400 to about 65,000, or from about 800 to about 50,000.

Preferred lower molecular weight silicone compounds useful herein include polyalkyl or polyaryl siloxanes with the following structure: 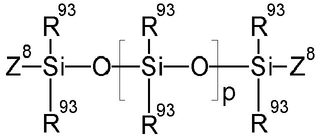 wherein R⁹³ is alkyl or aryl, and p is an integer from about 7 to about 850, more preferably from about 7 to about 665. Z⁸ represents groups which block the ends of the silicone chains. The alkyl or aryl groups substituted on the siloxane chain (R⁹³) or at the ends of the siloxane chains Z⁸ can have any structure as long as the resulting silicone remains fluid at room temperature, is dispersible, is neither irritating, toxic nor otherwise harmful when applied to the surface, is compatible with the other components of the aqueous composition, is chemically stable under normal use and storage conditions, and is capable of being deposited on and conditions the surface. Suitable Z⁸ groups include hydroxy, methyl, methoxy, ethoxy, propoxy, and aryloxy. The two R⁹³ groups on the silicon atom may represent the same group or different groups. Preferably, the two R⁹³ groups represent the same group. Suitable R⁹³ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. Commercially available these silicone compounds useful herein include, for example, those available from the General Electric Company in their TSF451 series, and those available from Dow Corning in their Dow Corning SH200 series.

The liquid benefit agent of the present invention may include one or more aminosilicones. Aminosilicones, as provided herein, are silicones containing at least one primary amine, secondary amine, tertiary amine, or a quaternary ammonium group. Preferred aminosilicones may have less than about 0.5% nitrogen by weight of the aminosilicone, more preferably less than about 0.2%, more preferably still, less than about 0.1%. Higher levels of nitrogen (amine functional groups) in the amino silicone tend to result in less friction reduction, and consequently less conditioning benefit from the aminosilicone. It should be understood that in some product forms, higher levels of nitrogen are acceptable in accordance with the present invention.

The aminosilicone may have a viscosity of from about 1,000 centipoise ("cPs") to about 100,000 cPs, or from about 2,000 cPs to about 50,000 cPs, or from about 4,000 cPs to about 40,000 cPs, or from about 6,000 cPs to about 30,000 cPs. The viscosity of aminosilicones discussed herein is measured at 25°C.

The aminosilicone can be contained in the aqueous composition of the present invention at a level by weight of from about 0.5% to about 30%, or from about 1.0% to about 24%, or from about 2.0% to about 16%, or from about 3.0% to about 8%.

Examples of preferred aminosilicones for use in the present invention include, but are not limited to, those which conform to the general formula (I):

(R¹)ₐG₃₋ₐ-Si-(-OSiG₂)ₙ-(-OSiG_{b}(R¹)_{2-b})ₘ-O-SiG₃₋ₐ(R¹)ₐ **(I)**

wherein G is hydrogen, phenyl, hydroxy, or C₁-C₈ alkyl, preferably methyl; a is 0 or an integer having a value from 1 to 3, preferably 1; b is 0, 1, or 2, preferably 1; wherein when a is 0, b is not 2; n is a number from 0 to 1,999; m is an integer from 0 to 1,999; the sum of n and m is a number from 1 to 2,000; a and m are not both 0; R¹ is a monovalent radical conforming to the general formula CqH_{2q}L, wherein q is an integer having a value from 2 to 8 and L is selected from the following groups: -N(R²)CH₂-CH₂-N(R²)₂; -N(R²)₂; -N(R²)⁺₃A⁻; -N(R²)CH₂-CH₂-N R²H₂A⁻; wherein R² is hydrogen, phenyl, benzyl, or a saturated hydrocarbon radical, preferably an alkyl radical from about C₁ to about C₂₀; A⁻ is a halide ion.

Some silicones for use herein can include those aminosilicones that correspond to formula (I) wherein m=0, a=1, q=3, G=methyl, n is preferably from about 1500 to about 1700, more preferably about 1600; and L is -N(CH₃)₂ or -NH₂, more preferably -NH₂. Other aminosilicones can include those corresponding to formula (I) wherein m=0, a=1, q=3, G=methyl, n is preferably from about 400 to about 600, more preferably about 500; and L is -N(CH₃)₂ or -NH₂, more preferably -NH₂. These aminosilicones can be called as terminal aminosilicones, as one or both ends of the silicone chain are terminated by nitrogen containing group.

An exemplary aminosilicone corresponding to formula (I) is the polymer known as "trimethylsilylamodimethicone", which is shown below in formula (II): 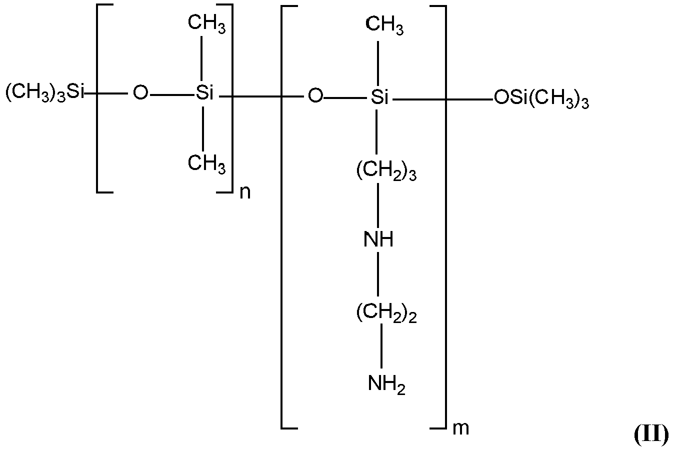 wherein n is a number from 1 to 1,999 and m is a number from 1 to 1,999.

The silicone may also be a terminal aminosilicone. "Terminal aminosilicone" as defined herein means a silicone polymer comprising one or more amino groups at one or both ends of the silicone backbone. The hydrophobic coating may be substantially free of any silicone compound other than terminal aminosilicones.

The amino group at least one terminus of the silicone backbone of the terminal aminosilicone may be selected from the group consisting of: primary amines, secondary amines and tertiary amines. The terminal aminosilicone may conform to Formula III:

(R₁)ₐG₃₋ₐ-Si-(-OSiG₂)ₙ-O-SiG₃₋ₐ(R₁)ₐ **(III)**

wherein G is hydrogen, phenyl, hydroxy, or C₁-C₈ alkyl, preferably methyl; a is an integer having a value from 1 to 3, or is 1; b is 0, 1 or 2, or is 1; n is a number from 0 to 1,999; R₁ is a monovalent radical conforming to the general formula CqH_{2q}L, wherein q is an integer having a value from 2 to 8 and L is selected from the following groups: -N(R²)CH₂-CH₂-N(R²)₂; -N(R²)₂; -N(R₂)₃A⁻; -N(R₂)CH₂-CH₂-NR₂H₂A⁻; wherein R₂ is hydrogen, phenyl, benzyl, or a saturated hydrocarbon radical; A⁻ is a halide ion. R₂ may be an alkyl radical having from 1 to 20 carbon atoms, or from 2 to 18 carbon atoms, or from 4 to 12 carbon atoms.

A suitable terminal aminosilicone corresponding to Formula III has a=1, q=3, G=methyl, n is from about 1000 to about 2500, alternatively from about 1500 to about 1700; and L is -N(CH₃)₂. A suitable terminal aminosilicone corresponding to Formula III has a=0, G=methyl, n is from about 100 to about 1500, or from about 200 to about, L is selected from the following groups: -N(R²)CH₂-CH₂-N(R²)₂; -N(R²)₂; -N(R₂)₃A⁻; -N(R₂)CH₂-CH₂-NR₂H₂A⁻; wherein R₂ is hydrogen, phenyl, benzyl, or a saturated hydrocarbon radical; A is a halide ion, alternatively L is -NH₂. R₂ may be an alkyl radical having from 1 to 20 carbon atoms, or from 2 to 18 carbon atoms, or from 4 to 12 carbon atoms. The terminal aminosilicone may be selected from the group consisting of bis-aminomethyl dimethicone, bis-aminoethyl dimethicone, bis-aminopropyl dimethicone, bis-aminobutyl dimethicone, and mixtures thereof.

Suitable terminal aminosilicones include aminopropyl terminated polydimethylsiloxane (e.g. having a viscosity of 4,000-6,000 cSt (4-6 Pa·s); available under the tradename DMS-A35 from Gelest, Inc.), polydimethylsiloxane, trimethylsiloxy terminated (e.g. having a viscosity of 5,000 cSt (5 Pa·s); available under the tradename DMS-T35 from Gelest, Inc.), polydimethylsiloxane, trimethylsiloxy terminated (e.g. having a viscosity of 1,000 cSt (1 Pa·s); available under the tradename DMS-T31 from Gelest, Inc.), aminopropyl terminated polydimethylsiloxane (e.g. having a viscosity of 900-1,100 cSt (0.9-1.1 Pa·s); available under the tradename DMS-A31 from Gelest, Inc.), polydimethylsiloxane, trimethylsiloxy terminated (e.g. having a viscosity of 50 cSt (0.05 Pa·s); available under the tradename DMS-T15 from Gelest, Inc.), aminopropyl terminated polydimethylsiloxane (e.g. having a viscosity of 50-60 cSt (0.05-0.06 Pa·s); available under the tradename DMS-A15 from Gelest, Inc.), bis-aminopropyl dimethicone (e.g. having a viscosity of 10,220 cSt (10.2 Pa·s); available from Momentive Performance Materials Inc.), and mixtures thereof.

### Alkyl Siloxane Polymer

Suitable conditioning agents as benefit agents of the hydrophobic coating further include alkyl siloxane polymers, as described in detail in US 2011/0243874 A1, US 2011/0243875 A1, US 2011/0240065 A1, US 2011/0243878A1, US 2011/0243871 A1, and US 2011/0243876 A1.

CATIONIC ORGANOPOLYSILOXANES Suitable conditioning agents as benefit agents of the hydrophobic coating further include cationic organopolysiloxanes, as described in detail in US 2014/0030206 A1, WO 2014/018985 A1, WO 2014/018986 A1, WO 2014/018987 A1, WO 2014/018988 A1, and WO 2014/018989 A1.

### Organic Oils

The liquid benefit agent of the present invention may comprise at least one organic conditioning oil as the conditioning agent, either alone or in combination with other conditioning agents, such as the silicones.

The hydrocarbon-based benefit material comprises an average carbon chain length of greater than 20, or an average carbon chain length of greater than 30, or an average carbon chain length of greater than 40.

### Hydrocarbon Oils

Suitable organic oils for use as liquid benefit agents in the aqueous compositions of the present invention include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from about C₁₂ to about C₁₉. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polyisobutylene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from Amoco Chemical Corporation. The concentration of such hydrocarbon oils in the aqueous composition can range from about 0.05% to about 20%, alternatively from about 0.08% to about 1.5%, and alternatively from about 0.1% to about 1%.

### Polyolefins

Organic oils for use as liquid benefit agents can also include liquid polyolefins, more preferably liquid poly-α-olefins, more preferably hydrogenated liquid poly-α-olefins. Polyolefins for use herein are prepared by polymerization of C₄ to about C₁₄ olefenic monomers, preferably from about C₆ to about C₁₂.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl- 1-pentene, and mixtures thereof. Also suitable for preparing the polyolefin liquids are olefin-containing refinery feedstocks or effluents. Preferred hydrogenated α-olefin monomers include, but are not limited to: 1-hexene to 1-hexadecenes, 1-octene to 1-tetradecene, and mixtures thereof.

### Fatty Esters

Other suitable organic oils for use as the conditioning agent in the aqueous compositions of the present invention include, but are not limited to, fatty esters having at least 10 carbon atoms. These fatty esters include esters with hydrocarbyl chains derived from fatty acids or alcohols (e.g. mono-esters, polyhydric alcohol esters, and di- and tri-carboxylic acid esters). The hydrocarbyl radicals of the fatty esters hereof may include or have covalently bonded thereto other compatible functionalities, such as amides and alkoxy moieties (e.g., ethoxy or ether linkages, etc.).

Specific examples of preferred fatty esters include, but are not limited to: isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the aqueous compositions of the present invention are monocarboxylic acid esters of the general formula R'COOR, wherein R' and R are alkyl or alkenyl radicals, and the sum of carbon atoms in R' and R is at least 10, preferably at least 22.

Still other fatty esters suitable for use in the aqueous compositions of the present invention are di- and tri-alkyl and alkenyl esters of carboxylic acids, such as esters of C₄ to C₈ dicarboxylic acids (e.g. C₁ to C₂₂ esters, preferably C₁ to C₆, of succinic acid, glutaric acid, and adipic acid). Specific non-limiting examples of di- and tri- alkyl and alkenyl esters of carboxylic acids include isocetyl stearyol stearate, diisopropyl adipate, and tristearyl citrate.

Other fatty esters suitable for use in the aqueous compositions of the present invention are those known as polyhydric alcohol esters. Such polyhydric alcohol esters include alkylene glycol esters, such as ethylene glycol mono and di-fatty acids, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Still other fatty esters suitable for use in the aqueous compositions of the present invention are glycerides, including, but not limited to, mono-, di-, and tri-glycerides, preferably di- and tri-glycerides, more preferably triglycerides. For use in the aqueous compositions described herein, the glycerides are preferably the mono-, di-, and tri-esters of glycerol and long chain carboxylic acids, such as C₁₀ to C₂₂ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

Other fatty esters suitable for use in the aqueous compositions of the present invention are water insoluble synthetic fatty esters. Some preferred synthetic esters conform to the general Formula (IX): 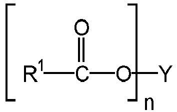 wherein R¹ is a C₇ to C₉ alkyl, alkenyl, hydroxyalkyl or hydroxyalkenyl group, preferably a saturated alkyl group, more preferably a saturated, linear, alkyl group; n is a positive integer having a value from 2 to 4, preferably 3; and Y is an alkyl, alkenyl, hydroxy or carboxy substituted alkyl or alkenyl, having from about 2 to about 20 carbon atoms, preferably from about 3 to about 14 carbon atoms. Other preferred synthetic esters conform to the general Formula (X): 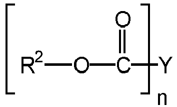 wherein R² is a C₈ to C₁₀ alkyl, alkenyl, hydroxyalkyl or hydroxyalkenyl group; preferably a saturated alkyl group, more preferably a saturated, linear, alkyl group; n and Y are as defined above in Formula (X).

Specific non-limiting examples of suitable synthetic fatty esters for use in the aqueous compositions of the present invention include: P-43 (C₈-C₁₀ triester of trimethylolpropane), MCP-684 (tetraester of 3,3 diethanol-1,5 pentadiol), MCP 121 (C₈-C₁₀ diester of adipic acid), all of which are available from Mobil Chemical Company.

### Metathesized Unsaturated Polyol Esters

Other suitable organic oils as benefit agents include metathesized unsaturated polyol esters. Exemplary metathesized unsaturated polyol esters and their starting materials are set forth in US 2009/0220443 A1. A metathesized unsaturated polyol ester refers to the product obtained when one or more unsaturated polyol ester ingredient(s) are subjected to a metathesis reaction. Metathesis is a catalytic reaction that involves the interchange of alkylidene units among compounds containing one or more double bonds (i.e., olefinic compounds) via the formation and cleavage of the carbon-carbon double bonds. Metathesis may occur between two of the same molecules (often referred to as self-metathesis) and/or it may occur between two different molecules (often referred to as cross-metathesis).

### Silane-Modified Oils

Other suitable organic oils as liquid benefit agents include silane-modified oils. In general, suitable silane-modified oils comprise a hydrocarbon chain selected from the group consisting of saturated oil, unsaturated oil, and mixtures thereof; and a hydrolysable silyl group covalently bonded to the hydrocarbon chain. Suitable silane-modified oils are described in detail in US Application Serial No. 61/821,818, filed May 10, 2013.

### Other Liquid Benefit Agents

Also suitable for use in the aqueous compositions herein are the liquid benefit agents described by the Procter & Gamble Company in U.S. Pat. Nos. 5,674,478, and 5,750,122. Also suitable for use herein are those liquid benefit agents described in U.S. Pat. Nos. 4,529,586 (Clairol), 4,507,280 (Clairol), 4,663,158 (Clairol), 4,197,865 (L'Oreal), 4,217, 914 (L'Oreal), 4,381,919 (L'Oreal), and 4,422, 853 (L'Oreal).

### Perfume

The hydrophobic benefit agent of the present invention may also include one or more perfumes. The one or more perfumes may be selected from any perfume or perfume chemical suitable for topical application to the particular surface to be treated. The concentration of the perfume in the personal care composition should be effective to provide the desired aroma including, but not limited to, unscented. Generally, the concentration of the scented primary perfume is from about 0.5% to about 30%, or from about 1% to about 20%, or from about 2% to about 10%, or from about 3% to about 8%, by weight of the solid article.

The perfume may be selected from the group consisting of perfumes, highly volatile perfume materials having a boiling point of less than about 250°C, and mixtures thereof. The perfume may be selected from high impact accord perfume ingredients having a ClogP of greater than about 2 and odor detection thresholds of less than or equal to 50 parts per billion (ppb).

### Aqueous carrier

The aqueous composition may include an aqueous carrier. The aqueous carrier which is used may be distilled, deionized, or tap water. Water may be present in any amount for the composition to be an aqueous solution. Water may be present in an amount of about 85 wt. % to 99.5 wt. %, preferably about 90 wt. % to about 99.5 wt. %, more preferably about 92 wt. % to about 99.5 wt. %, more preferably about 95 wt. %, by weight of the composition. Water containing a small amount of low molecular weight monohydric alcohols, e.g., ethanol, methanol, and isopropanol, or polyols, such as ethylene glycol and propylene glycol, can also be useful.

### Minor Ingredients

The composition may include one or more minor ingredients in the aqueous phase. The minor ingredients are selected from the group consisting of: buffering agents, solubilizing agents, antimicrobial compounds, preservatives, malodor counteractants, perfume delivery technologies, diluents, antioxidants, water soluble metallic salts, including zinc salts, copper salts, antistatic agents; insect repelling agents; colorants, and combinations thereof.

The aqueous composition may contain a substantially low level of a solubilizing aid to solubilize any excess hydrophobic organic materials, particularly some malodor reduction materials, perfume materials, and also optional ingredients (e.g., insect repelling agent, antioxidant, etc.) which can be added to the aqueous composition, that are not readily soluble in the aqueous composition, to form a clear translucent solution. A suitable solubilizing aid is a surfactant, such as a no-foaming or low-foaming surfactant. Suitable surfactants are nonionic surfactants, cationic surfactants, amphoteric surfactants, zwitterionic surfactants, and mixtures thereof. If present, it is preferred that the solubilizing aid be a non-ionic surfactant.

The aqueous composition may contain nonionic surfactants, cationic surfactants, and mixtures thereof. The aqueous composition may contain ethoxylated hydrogenated castor oil. One type of suitable hydrogenated castor oil that may be used in the aqueous composition is sold as Basophor™, available from BASF.

If the aqueous composition comprises surfactant, the total amount of surfactant present in the aqueous composition is less than 10,000 ppm, preferably less than 1,000 ppm and most preferably less than 100 ppm. The aqueous composition may be substantially free of a surfactant. Or, the aqueous composition may not comprise an effective amount of surfactant. As used herein, an "effective amount of surfactant" is an amount of surfactant in an aqueous composition at which the surfactant behaves as an emulsifier between the liquid benefit agent droplets and the aqueous carrier.

Buffering agents may include carboxylic acid, a dicarboxylic acid such as maleic acid, or a polybasic acid such as citric acid, or polyacrylic acid.

Nonlimiting examples of surface-tension reducing agents of this type are described in US 5,714,137 and include the Silwet* surfactants available from Momentive Performance Chemical, Aliquid benefit agentny, New York. Exemplary Silwet surfactants are as follows:

| Name | Average MW |
|---|---|
| L-7608 | 600 |
| L-7607 | 1,000 |
| L-77 | 600 |
| L-7605 | 6,000 |
| L-7604 | 4,000 |
| L-7600 | 4,000 |
| L-7657 | 5,000 |
| L-7602 | 3,000 |

Water-soluble antimicrobial compounds include organic sulfur compounds, halogenated compounds, cyclic organic nitrogen compounds, low molecular weight aldehydes, quaternary compounds, dehydroacetic acid, phenyl and phenoxy compounds, or mixtures thereof.

A quaternary compound may be used. Examples of commercially available quaternary compounds suitable for use in the aqueous composition are Barquat available from Lonza Corporation; and didecyl dimethyl ammonium chloride quat under the trade name Bardac® 2250 from Lonza Corporation.

The perfume delivery technologies may be selected from the group consisting of: pro-perfumes, polymer particles, soluble silicone, polymer assisted delivery, molecule assisted delivery, fiber assisted delivery, amine assisted delivery, cyclodextrins, starch encapsulated accord, zeolite and inorganic carrier, and mixtures thereof.

### Solid Particles

The aqueous composition may include a plurality of solid particles. The solid particles may be in the form of mesoporous particles, activated carbon, zeolites, benefit agent delivery particle, wax, hydrogel, ground nutshells, and/or combinations thereof.

The solid particles may be in the form of benefit agent delivery particles. The benefit agent delivery particles may include a wall material that encapsulates a benefit agent. Benefit agent may be referred herein as a "benefit agent" or an "encapsulated benefit agent". The benefit agent may be selected from the group consisting of: a perfume mixture, an insect repellent, a malodor counteractant, and combinations thereof. The benefit agent may include materials selected from the group consisting of perfume raw materials such as 3-(4-t-butylphenyl)-2-methyl propanal, 3-(4-t-butylphenyl)-propanal, 3-(4-isopropylphenyl)-2-methylpropanal, 3-(3,4-methylenedioxyphenyl)-2-methylpropanal, and 2,6-dimethyl-5-heptenal, alpha-damascone, beta-damascone, gamma-damascone, beta-damascenone, 6,7-dihydro-1,1,2,3,3-pentamethyl-4(5H)-indanone, methyl-7,3-dihydro-2H-1,5-benzodioxepine-3-one, 2-[2-(4-methyl-3-cyclohexenyl-1-yl)propyl]cyclopentan-2-one, 2-sec-butylcyclohexanone, and beta-dihydro ionone, linalool, ethyllinalool, tetrahydrolinalool, and dihydromyrcenol; silicone oils, waxes such as polyethylene waxes; essential oils such as fish oils, jasmine, camphor, lavender; skin coolants such as menthol, methyl lactate; vitamins such as Vitamin A and E; sunscreens; glycerine; catalysts such as manganese catalysts or bleach catalysts; bleach particles such as perborates; silicon dioxide particles; antiperspirant actives; cationic polymers and mixtures thereof. Suitable benefit agents can be obtained from Givaudan Corp. of Mount Olive, New Jersey, USA, International Flavors & Fragrances Corp. of South Brunswick, New Jersey, USA, or Firmenich Company of Geneva, Switzerland.

The benefit agent could include materials that are liquid benefit agents, as described in the present invention. In such an example, an aqueous composition may include liquid benefit agent droplets discontinuously dispersed throughout the aqueous phase in addition to encapsulated liquid benefit agents that are also dispersed throughout the aqueous phase.

In one aspect, the perfume delivery technology may comprise benefit agent delivery particles formed by at least partially surrounding a benefit agent with a wall material.

The wall material of the benefit agent delivery particle may comprise: melamine, polyacrylamide, silicones, silica, polystyrene, polyurea, polyurethanes, polyacrylate based materials, polyacrylate esters based materials, gelatin, styrene malic anhydride, polyamides, aromatic alcohols, polyvinyl alcohol and mixtures thereof. The melamine wall material may comprise melamine crosslinked with formaldehyde, melamine-dimethoxyethanol crosslinked with formaldehyde, and mixtures thereof. The polystyrene wall material may comprise polyestyrene cross-linked with divinylbenzene. The polyurea wall material may comprise urea crosslinked with formaldehyde, urea crosslinked with gluteraldehyde, polyisocyanate reacted with a polyamine, a polyamine reacted with an aldehyde and mixtures thereof. The polyacrylate based wall materials may comprise polyacrylate formed from methylmethacrylate/dimethylaminomethyl methacrylate, polyacrylate formed from amine acrylate and/or methacrylate and strong acid, polyacrylate formed from carboxylic acid acrylate and/or methacrylate monomer and strong base, polyacrylate formed from an amine acrylate and/or methacrylate monomer and a carboxylic acid acrylate and/or carboxylic acid methacrylate monomer, and mixtures thereof.

The polyacrylate ester-based wall materials may comprise polyacrylate esters formed by alkyl and/or glycidyl esters of acrylic acid and/or methacrylic acid, acrylic acid esters and/or methacrylic acid esters which carry hydroxyl and/or carboxy groups, and allylgluconamide, and mixtures thereof.

The aqueous composition may comprise any amount of particles. With regard to benefit agent delivery particles, the aqueous composition may contain from about 0.001 wt. % to about 2.0 wt. %, by weight of aqueous composition, of benefit agent contained with the wall material of the benefit agent delivery particle. Or, the aqueous composition may contain from about 0.01 wt. % to about 1.0 wt. %, or most preferably from about 0.05 wt. % to about 0.5 wt. %, by weight of aqueous composition, of benefit agent contained with the wall material of the benefit agent delivery particle.

The aqueous composition of the present invention may be a hair conditioner composition. The hair conditioner composition may contain one or more liquid benefit agents that are water-insoluble hydrophobic conditioning agents such as silicones, organic oils or other water-insoluble conditioning agents.

The hair conditioner composition may also contain a gel matrix comprising a combination of a cationic surfactant and a high melting point fatty alcohol (melting point higher than 25oC).

Non-limiting examples of high melting fatty alcohols include etyl alcohol, stearyl alcohol, behenyl alcohol, and mixtures thereof. An example hair conditioning composition of the present invention is shown in Example 1.

| Ingredients | Example 1 |
|---|---|
| Behentrimonium Chloride (wt% active) | 3.5 |
| Cetyl Alcohol (wt% active) | 0.87 |
| Stearyl Alcohol (wt% active) | 2.6 |
| Aminosilicone | 0.50 |
| Xanthan gum | 0.04 |
| Tara gum | 0.04 |
| Disodium EDTA | 0.10 |
| Benzyl Alcohol | 0.40 |
| Preservative | 0.0005 |
| Water | Q.S. |

### METHOD OF MAKING

As discussed above, it has been found that the method of making the aqueous composition of the present invention results in a phase-stable aqueous composition that comprises an aqueous phase comprising a structurant system and liquid benefit agent droplets discontinuously dispersed throughout the aqueous phase. Forming a phase-stable discontinuous phase of liquid benefit agent droplets in final aqueous composition requires at least two steps: and Emulsification Step and Dilution Step.

In the emulsification step, the liquid benefit agent is emulsified into a concentrate. The concentrate comprises some or all the structurant system, some water and optionally part or all of the minors. The concentrate is first formed by mixing the structurant system, water, and any minors. Then, the liquid benefit agent is emulsified into the concentrate with the aid of aggressive mixing. Not wishing to be bound by theory, the concentrate has a relatively high concentration of structurant system set the viscosity to substantially match the viscosity of the liquid benefit agent in order to effectively disperse the liquid benefit agent. Further, the ratio and concentration of the polysaccharide is selected to ensure the presence of a yield stress which eliminates the drainage between droplets in the concentrate ensuring stability versus creaming and coalescence of the droplets. Both considerations are necessary, to surprisingly allow the formation of stable emulsions of liquid benefit agent droplets without the need of surfactant or with a very low level of surfactant.

In the dilution step, the concentrate comprising the liquid benefit agent droplets is then diluted with additional water to form the final aqueous composition. Along with the additional water, additional structurant system and/or minor ingredients may also be added to the concentrated aqueous phase. Subsequently diluting the concentrate has been found to not affect the stable suspension of liquid benefit agent droplets formed in the concentrate of the aqueous phase.

A variety of mixers may be used to mix the liquid benefit agent into the concentrated aqueous phase. The mixer may be a high-speed mixer, static mixer, overhead mixer, or cetera mixer. Preferably a high-speed mixer may be used to form liquid benefit agent droplets. However, any mixer is suitable provided it applies enough mixing to disperse the liquid benefit agent into liquid benefit agent droplets.

In order to emulsify the liquid benefit agents in the concentrate, the viscosity of concentrate may be between 10 Pa·s and 0.00 Pa·s, more preferably between 1 Pa·s - 0.01 Pa·s, more preferably between 1 Pa·s - 0.1 Pa·s and most preferably between 1 Pa·s - 0.3 Pa·s, as determined by the RHEOLOGY TEST METHOD. The concentrate must exhibit a yield stress greater than 2 Pa - 0 Pa, more preferably between 1.5 Pa - 0.02 Pa, more preferably between 1.0 Pa - 0.05 Pa and most preferably between 1 Pa - 0.3 Pa, as determined by the RHEOLOGY TEST METHOD.

The aqueous composition comprises between 0.1 wt. % to 20 wt. %, more preferably between 10 % - 0.2 %, more preferably between 5 % - 0.5 % and most preferably between 5 % - 0.5 % of the liquid benefit agent droplets
Success in the emulsification step is defined by liquid benefit agent droplet size, liquid benefit agent on surface of preparation and stability. The most preferred aqueous compositions have liquid benefit agent droplet diameters less than about 500 µm, most preferably less than about 250 µm and most preferably less than about 100 µm, as evident by haziness in the preparation; there is only a small amount essentially no liquid benefit agent on the surface of the preparation with 24 hours of making; there is no instabilities in the preparation, as indicated by bulk separation of the liquid benefit agent. The preferred aqueous compositions have liquid benefit agent droplet diameters less than about 500 µm, most preferably less than about 250 µm and most preferably less than about 100 µm, as evident by haziness in the preparation; there is only a small amount of liquid benefit agent on the surface of the preparation with 24 hours of making (estimated less than 5 wt% of total in mixture); there is no instabilities in the preparation, as indicated by bulk separation of the liquid benefit agent. The comparative embodiments have liquid benefit agent droplet diameters greater than about 500 µm or indications incomplete dispersion as evident by relative clarity in the preparation; there is significant liquid benefit agent on the surface of the preparation with 24 hours of making (estimated greater than 5 wt% of total in mixture); there is significant instabilities in the preparation, as indicated by bulk separation of the liquid benefit agent. Success in the dilution step carries the liquid benefit agent droplets with comparable droplet sizes into the most preferred or preferred aqueous composition.

### TEST METHODS

### RHEOLOGY TEST METHOD

To measure the yield stress and/or the viscosity of a sample (e.g. concentrate or aqueous composition, but not LBA), measurements are made with a TA Discovery HR-2 Hybrid Rheometer (TA Instruments, New Castle, Delaware, U.S.A.) and accompanying TRIOS software version 4.2.1.36612, or equivalent. The instrument is outfitted with a Concentric Cylinder Double Gap Cup (e.g., TA Instrument, cat. # 546050.901), Double Gap Rotor (e.g. TA Instruments, cat. # 546049.901) and Split Cover (e.g. TA Instruments, cat. # 545626.001). The calibration is done in accordance with manufacturer recommendations. A refrigerated, circulating water bath set to 25 °C is attached to the Concentric Cylinder. The Concentric Cylinder temperature is set to 25°C. The temperature is monitored within the Control Panel until the instrument reaches the set temperature, then an additional 5 minutes is allowed to elapse to ensure equilibration before loading sample material into the Double Gap Cup.

The parameters for the Double Gap Cup are as follows: the inside cup diameter is 30.2 mm; the inside bob diameter is 32 mm; the outside bob diameter is 35 mm; the outside cup diameter is 37 mm; the inner cylinder height is 55 mm; the immersed height is 53 mm; the operating gap is 2,000.0 µm; the loading gap is 90,000.0 µm; the Environmental system is Peltier; and with a sample volume between 12 ml and 15 ml (preferably 12 ml).

To load the sample, a minimum of 12 ml of sample is added to the Double Gap Cup using a syringe, and the sample is then allowed to sit for 15 minutes, ensuring that any trapped air bubbles rise to the surface. The Double Gap Rotor is then lowered to the proper gap and data are collected in accordance with the following Settings and Procedures.

Data are collected in a series of steps conducted in precisely the following order: The Conditioning Sample Step is conducted using the following instrumental settings: Environmental Control is set with a Temperature of 25 °C; Inherit Set Point is selected as Off; Soak Time is set to 0.0 s; Wait for Temperature is selected as On; Wait for axial force is selected as Off; Preshear Options is set with a Perform Preshear selected as Off; Equilibrium is set with a Perform Equilibration selected as On; and Duration is set to 600.0 s.

The Flow Peak Hold Step is conducted using the following instrument settings: Environmental Control is set with a Temperature of 25 °C; Inherit Set Point is selected as Off; Soak Time is set to 0.0 s; Wait for Temperature is selected as Off; Test Parameters is set with a Duration of 600.0 s; Shear Rate is selected and set to 0.01 s-1; Inherit initial value is selected as Off; Sampling interval is selected and set to 3.0 s/pt; Controlled Rate Advanced is set with a Motor mode selected as Auto; Data acquisition is set with a End of step selected as Zero torque; Fast sampling is selected as Off; Save image is selected as Off; Step Termination is set with Limit checking Enabled selected as On; Terminate step when is set with Strain(%) selected, > selected, and set to 500 %; Equilibrium Enabled is selected as Off; Step Repeat Enabled is selected as off.

The Conditioning Sample Step is conducted using the following instrumental settings: Environmental Control is set with a Temperature of 25 °C; Inherit Set Point is selected as Off; Soak Time is set to 10.0 s; Wait for Temperature is selected as Off; Wait for axial force is selected as Off; Preshear Options is set with a Perform Preshear selected as Off; Equilibrium is set with a Perform Equilibration selected as On; and Duration is set to 600.0 s.

The Flow Sweep Step is conducted using the following instrument settings: Environmental Control is set with a Temperature of 25 °C; Inherit Set Point is selected as Off; Soak Time is set to 0.0 s; Wait For Temperature is selected as Off; Test Parameters is set with Logarithmic sweep selected; Shear rate is selected and set to 1.0e-3 s-1 to 1000.0 s-1; Points per decade is set to 5; Steady state sensing is selected as On; Max equilibration time is set to 45.0 s; Sample period is set to 5.0 s; % tolerance is set to 5.0; Consecutive within is set to 3; Scaled time average is selected as Off; Controlled Rate Advanced is set with Motor mode selected as Auto; Data acquisition is set with Save point display selected as Off; Save image is selected as Off; Step termination is set with Limit checking Enabled selected as Off; Equilibrium Enabled is selected as Off; Step Repeat Enabled is selected as Off.

The Conditioning End of Test Step is conducted using the following instrument settings: Set temperature is selected as Off; Set temperature system idle (only if axial force control is active) is selected as On.

The Yield Stress is calculated from the data collected in the Flow Peak Hold Step, in the following way: The data points are plotted as Stress (mPa) on the y-axis against Step Time (s) on the x-axis. The Yield Stress is determined by selecting the "Analysis" tab, then selecting "Signal max" from the Function drop down list and finally selecting "Analyze" in the Commands category. For a contiguous data set (containing a single stress value greater than zero for each time value), the Yield Stress equals the value of 'Max Y' if it occurs in the first 250 s and the Yield Stress equals zero if the value of 'Max Y' occurs after 250 s. If the measured sample is a concentrate, the value is defined as the yield stress of the concentrate ('YS_{conc.}'); if the measured sample is an aqueous composition, the value is defined as the yield stress of the aqueous composition ('YS_{aqu.}'); the yield stress of the concentrate and/or aqueous composition is assigned a value of 'NM' if the yield stress was not measured.

The Viscosity is determined to be the viscosity measured when the shear rate is 10 s-1, or measured at the nearest data point within 1% of 10 s-1 shear rate, expressed in mPa·s. If the measured sample is a concentrate, the value is defined as the viscosity of the concentrate ('h_{conc.}'); if the measured sample is an aqueous composition, the value is defined as the viscosity of the aqueous composition ('h_{aqu.}'); the viscosity of the concentrate and/or aqueous composition is assigned a value of 'NM' if the yield stress was not measured.

### VISCOSITY OF LBA TEST METHOD

Viscosity measurements of the liquid benefit agent are made with a TA Discovery HR-2 Hybrid Rheometer (TA Instruments, New Castle, Delaware, U.S.A.) and accompanying TRIOS software version 4.2.1.36612, or equivalent. The instrument is outfitted with a 60 mm stainless steel Cone with a 2-degree angle (e.g., TA Instruments, cat. # 511606.905) Peltier plate (e.g., TA Instruments cat. # 533230.901). The calibration is done in accordance with manufacturer recommendations. A refrigerated, circulating water bath set to 25 °C is attached to the Peltier plate. The Peltier Plate temperature is set to 25 °C. The temperature is monitored within the Control Panel until the instrument reaches the set temperature.

To load liquid benefit agent (LBA), a 5 ml slip tip syringe or similar, is used to transfer 2 ml of LBA onto the center surface of the Peltier plate. If the loaded sample liquid contains visible bubbles, a period of 10 minutes is waited to allow the bubbles to migrate through the sample and burst, or a transfer pipette can be used to extract the bubbles. If visible bubbles still remain, then the sample is removed from the Plate, the plate is cleaned with isopropanol wipe and the solvent is allowed to evaporate away. The sample loading procedure is then attempted again and repeated until a sample is loaded successfully without containing visible bubbles.

The 60 mm cone is lowered to the "trim gap", which for this attachment is specific to the cone and indicated to be 66 micrometers. The cone is then locked, any excess sample material is removed from the perimeter of the cone using flexible rubber spatula such as a rubber policeman. It is important to ensure that the sample is evenly distributed around the edge of the cone and there is no sample on the side or top of plate. If there is sample material on the side or top of the cone, this excess material is gentle removed. If there is sample material on the side or top of the plate, this excess material is gentle removed. The Solvent Trap Cover is carefully applied over the cone, and the cone is lowered into its final position by setting the gap distance to 55 micrometers.

Data are collected in a series of steps conducted in precisely the following order: The Conditioning Sample Step is conducted using the following instrumental settings: Environmental Control is set with a Temperature of 25 °C; Inherit Set Point is selected as Off; Soak Time is set to 0.0 s; Wait for Temperature is selected as On; Wait for Axial Force is selected as Off; Preshear Options is set with a Perform Preshear selected as On; Shear rate is set to 100.0 1/s; Duration is set to 20.0 s; Advanced options are set with Zero speed after pre-shear selected to be Leave as is; Zero velocity threshold set as 0.1 rad/s; Motor mode is selected as Auto; Equilibrium is set with a Perform Equilibration selected as On; and Duration is set to 600.0 s.

The Flow Sweep Step is conducted using the following instrument settings: Environmental Control is set with a Temperature of 25 °C; Inherit Set Point is selected as Off; Soak Time is set to 0.0 s; Wait For Temperature is selected as Off; Test Parameters is set with Logarithmic sweep selected; Shear rate is selected and set to 1.0e-3 s-1 to 1000.0 s-1; Points per Decade is set to 15; Steady state sensing is selected as On; Max Equilibration time is set to 45.0 s; Sample period is set to 5.0 s; % Tolerance is set to 5.0; Consecutive Within is set to 3; Scaled Time Average is selected as Off; Controlled Rate Advanced is set with Motor mode selected as Auto; Data Acquisition is set with Save point display selected as Off; Save image is selected as Off; Step Termination is set with Limit checking Enabled selected as Off; Equilibrium Enabled is selected as Off; Step Repeat Enabled is selected as Off.

The Conditioning End of Test Step is conducted using the following instrument settings: set temperature is selected as On; Temperature is set as 25 °C; Set temperature system idle (only if axial force control is active) is selected as Off.

The LBA Viscosity is determined to be the viscosity measured when the shear rate is at 100 s-1, or measured at the nearest data point within 1% of the 100 s-1 shear rate. The reported Viscosity Value for the LBA ('h_{LBA}') is the average of the viscosity values obtained from three independent viscosity measurements (i.e. three replicate sample preparations), and is expressed in units of mPa·s; the reported Viscosity Value for the LBA ('h_{LBA}') is given the value of 'NM' if the viscosity of the sample was not measured.

### DROP SIZE TEST METHOD

An optical microscope is used to determine the drop size, which is the number-weighted mean diameter of liquid benefit agent droplets in test samples of the composition. Note that the drop size values claimed herein refer to the mean diameter and that the values are number-weighted, not volume-weighted. The droplets are imaged using a transmitted-light compound microscope, equipped with a digital camera and an array of flat-field objective lenses ranging from 10x to 100x in magnification. The microscope is aligned for Kohler illumination and is calibrated for linear measurements in the x-y image plane using a stage micrometer. Suitable microscopes include the Zeiss Axio Imager (Carl Zeiss AG, Oberkochen, Germany) employing Normarski differential interference contrast (DIC) and equipped with a Zeiss AxioCam digital camera (Carl Zeiss AG, Oberkochen, Germany), or equivalent. One skilled in the art may choose to use image analysis software (such as AxioVision, Carl Zeiss Microscopy GmbH, Germany, or Image-Pro Premier, Media Cybernetics, Rockville Maryland USA, or equivalent) to measure the diameter of imaged droplets. Measurements within the images may also be achieved using accurately calibrated physical devices such as a ruler, caliper, reticule, graticule or similar.

Prior to determining their mean drop size, samples must be visually examined to confirm that they are well emulsified comprising a homogeneous dispersion of LBA. Samples that are not well emulsified may exhibit one of the following two characteristics: 1) comprise a few, very large drops of LBA such that the sample appears almost clear (i.e., little turbidity), or 2) comprise a concentration of LBA that is visibly enriched or increased near the surface of the preparation, rather than the LBA being dispersed uniformly throughout the volume. If the sample appears visually well emulsified, then the mean drop size is measured as described below. If the sample does not appear to be well emulsified, then the mean drop size is not measured and instead the final mean drop size diameter value is assigned a value of 'IE' for Ineffective Emulsion."

Samples of a well emulsified composition are used to create wet-mount slide preparations, wherein several drops of composition are placed on a standard or concavity glass microscope slide and gently covered with a standard glass coverslip. Wet-mount slide preparations are created in triplicate. The preparations are viewed microscopically without delay, and with care taken to place as little pressure and shear on the coverslip as is possible.

The prepared samples are observed microscopically under several different magnifications to assess the range of droplet sizes that are typical and numerically most common in the sample. This assessment is used to select objective lens that provide images wherein the diameters of the most common representative droplets are accommodated fully within the captured images and are also magnified sufficiently to permit accurate measurement of their diameter. Such images are captured until at least 10 representative droplets have been photographed from each replicate sample preparation.

Within the captured images, the diameter is measured of every particle which appears to be an approximately circular (spherical) droplet. Measurements of non-droplet objects, such as air bubbles or solid microcapsules, are excluded from the recorded values. The mean diameter value is calculated from all the droplets measured in each replicate wet-mount slide preparation. The final mean diameter value ('*D̅* _{LBA}') is the mean value calculated across all replicates and is reported in micrometers as the liquid benefit agent drop size diameter. The final mean diameter value ('*D̅* _{LBA}') is given a value of 'NM' if the mean drop size was not measured on the sample.

### SPRAYABLE TEST METHOD

The sprayability of a composition is determined by measuring the angle of the spray cone that is produced during attempted spraying, and comparing this value to that obtained from the negative control reference solution specified. Compositions are classified as either "Sprayable" or "Unsprayable". Compositions typically form a visible cone-shaped plume of droplets exiting the nozzle, wherein the cone is narrowest nearest to the nozzle and becomes wider as it extends further away from the nozzle, before the plume of droplets ultimately disperses. The spray cone angle is measured by attempting to spray the composition perpendicularly across the viewing area of a recording video camera under well-lit and non-drafty conditions. The images captured by the video camera are analyzed to determine the cone angle of the visible plume of sprayed material, within the region that extends 10 cm outward from the nozzle in the direction of the plume's trajectory. The spray cone angle is defined as the angle measured between two specified straight lines. One of the specified straight-lines is the central axis of the plume's trajectory away from the nozzle, while the other specified straight line is the plume's visible upper outer margin as described by its straight-line average across the first 10 cm of distance away from the nozzle.

The video camera is securely mounted to prevent movement or vibration, and is positioned, zoomed, and focused such that the plume region extending at least 10 cm from the nozzle is in sharp focus. One suitable video camera is the Phantom V310 (Vision Research). Lighting and backdrop conditions are selected such that the plume of droplets is easily observed with high contrast in the captured images. The composition, bottle, and environment are at the ambient laboratory air temperature of between 18 °C and 25 °C. The air conditions surrounding the nozzle and spray plume region are as still and stationary as possible.

During the analysis, each composition is dispensed from a bottle as a spray plume. The composition is loaded in small hand-held atomizer spray bottles having a top push-button pump activation mechanism. Such bottles include 10 mL volume refillable bottles which are commonly used by travelers to dispense perfumes or other sprayable cosmetic fluids. Suitable bottles include Dropper Stop, 10ml Aluminum Silver Color Travel Sprayer. Samples are test sprayed several times immediately prior to being imaged by the camera, in order to confirm that the bottle tube is primed and that the nozzle is not blocked. During testing, the spray actuator button is depressed manually with an index finger using consistent force and speed such that the duration of the full actuating stoke is as fast as possible and less than 1 second in length. Suitable interval speed includes synchronous compression with a 90 sec⁻¹ metronome, with full compression on the beat.

During the analysis, each test composition is dispensed from a bottle as a spray plume. Two standard control solutions are also dispensed and their spray cone angle measured, which serve as reference values demonstrating sprayable versus unsprayable performance. The negative control reference solution is a 0.1 wt % aqueous solution of 1 million Dalton PEO polymer (e.g., Aldrich 372781-250G), which exemplifies an unacceptable / unsprayable performance. The positive control reference solution is deionized water, which exemplifies an acceptable / sprayable performance. A composition is given a Spray Grade of 'Sprayable' if the spray angle is significantly larger than negative control, such that [(spray angle composition - spray angle negative control) / (spray angle positive control - spray angle negative control)] > 0.10. Otherwise a composition is given a Spray Grade of 'Unsprayable'. For the example data provided herein, a composition is given a Spray Grade of 'NM' if the spray angle measurement was not conducted on the sample.

### POLYSACCHARIDE WEIGHT-AVERAGE MOLECULAR WEIGHT TEST METHOD

Gel Permeation Chromatography with Multi-Angle Light Scattering Detection (GPC-MALS) is used to measure the weight average molecular weight (Mw) of xanthan gum. A suitable instrument is a Waters 2695 Separation Module (Waters Associates), with a DAWN EOS 18-angle LS and Optilab REX differential RI detectors (Wyatt Technology) connected in series, or equivalent equipment.

Solutions of the gum were prepared by dispersing approximately 10 mg of gum materials in 5 mL of purified HPLC grade water. Sample solutions were mixed and allowed to swell overnight. Each sample was diluted to the concentration of 0.2 mg/ml with 0.1M NaNO3 buffer before the GPC-MALS analysis. Samples were filtered directly into HPLC vials through 0.45 µm Nylon 66 filters (Thermo Fisher Scientific) to remove any microgels or particulate matter.

The separation is performed on two GPC columns such as Waters 7.8 x 300 mm Ultrahydrogel 2000 and 7.8 x 300 mm Ultrahydrogel 250 connected in series, or equivalent columns, maintained at 40 °C. Components are eluted with a mobile phase of 0.1M NaNO3 at an isocratic flow rate of 1.00 mL/min. 50 µL of the sample is injected for analysis.

Data from the two detectors is collected digitally using suitable software (e.g. Wyatt Astra software). The weight average molecular weight (Mw) is calculated using the Zimm equation. A refractive index increment (dn/dc) of 0.145 ml/g, typical for xanthan gum in aqueous solutions, was used in the calculation.

### POLYMER ACETYLATION TEST METHOD

Flow Injection Electrospray Ionization Quadrupole Time-Of-Flight Mass Spectrometry (FI-QTOF-MS) is used to measure the ratio of the acetylated sugar to non-acetylated sugar fragments, and thus determine the relative degree of acetylation modification in the polysaccharide polymer material being tested. In this tandem mass spectrometry analysis the first quadrupole mass analyzer is set at the RF only (broad band) mode so that all ions generated by the electrospray ionization are passed through this first mass filter without selection of a specific precursor ion. All ions after passing the first quadrupole are then fragmented in the second quadrupole. All fragment ions are finally mass separated and detected by the TOF mass analyzer according to their mass-to-charge ratio (m/z). For polymer or gum materials, polysaccharides are fragmented to give structure signature ion m/z 205 (the acetylated sugar) and structure signature ion m/z 163 (the non-acetylated sugar). The ratio of the peak intensity values from these two mass fragments indicates the degree of acetylation present in the material being tested, wherein a higher ratio indicates a higher degree of acetylation.

A sample of any individual polymer raw material to be tested (e.g., a konjac flour, or a xanthan gum) is dissolved at a concentration of 0.5 mg of polymer into 1 ml of water. The aqueous polymer sample solution is mixed and allowed to hydrate overnight. After overnight hydration, the solution is filtered through 0.8 µm pore-size filters (e.g., the Versapor acrylic copolymer membrane disc filter from Pall Corporation of Port Washington New York USA, or equivalent). The filtered solution is placed in an HPLC sample vial for analysis.

Flow Injection-QTOF-MS analysis is conducted using a suitable tandem quadrupole mass spectrometer system with electrospray ionization (such as Q-Tof 2 instruments from the Waters Corporation of Milford Massachusetts USA, or equivalent). The accompanying software supplied by the instrument manufacturer (such as the Mass Lynx NT version 4.1 data acquisition and processing software from the Waters Corporation, or equivalent) is used to control the instrument and conduct the analysis. The instrumental system is configured with a delivery solvent of 5 mM ammonium acetate / 10 % acetonitrile in water at a rate of 40 µL/min. No columns are used in the instrument configuration. The electrospray capillary voltage is set at 3.5 kV. The first quadrupole mass analyzer is set at the RF only (broad band) mode. To fragment the polymer materials, the second quadrupole with the collision cell energy is set at 70 V so that both signature fragment ions, namely: m/z 205, and m/z 163, are generated. The TOF mass analyzer is scanned from 50 Da to 3000 Da, with a 5 min data acquisition time for each flow injection run. The peak intensity of the m/z 205 fragment (i.e., the acetylated sugar) and the peak intensity of m/z 163 fragment (i.e., the non-acetylated sugar) are acquired. The ratio of these two peak intensity values is calculated and represents the acetylated-to-nonacetylated sugar fragments ratio. This ratio indicates the relative degree of acetylation in the gum or polysaccharide polymer material being tested.

### EXAMPLES

### Example A

An inventive sample that demonstrates the preparation of a fabric treatment composition, with a very low viscosity perfume oil liquid benefit agent (Example A). The perfume oil is emulsified and stabilized in a product concentrate. Then, the product concentrate is diluted with water to form a sprayable aqueous composition.

### Materials

Ethanol (94.3%, Equistar Chemicals)
Alpha Cyclodextrin (Sigma Aldrich Product code C4642)
Diethylene Glycol (99.6%, Indorama Ventures LLC)
Lactic Acid, at 30% active (Purac PF 90, Purac Bioquimica SA) diluted to 30%
Koralone B119 (The Dow Chemical Company)
Konjac Gum (Nutricol® XP 3464, FMC Corp)
Xanthan Gum (Jungbunzlauer Inc.)
Hydroxypropyl Beta Cyclodextrin (Cavasol W7 HP TL, 40%, Wacker Biosolutions)
Sodium Hydroxide (50% Membrane Grade Brenntag Mid-South, Inc), diluted to 5 %;
Cardamom Ginger Lemongrass Natural Fragrance (Procter & Gamble Company)
Water (Millipore, 18MW)

### Preparation

### 1 wt % Xanthan Gum Stock Solution

494.61 grams of Water were added to a clean mixing vessel (1-liter glass beaker, VWR). 0.39 grams Koralone B-119 were added to the beaker, and stirred until homogeneously mixed. 5.0 grams Xanthan Gum were added quickly into the beaker while mixing at 5000 RPM using the Ross Mill Mixer fitted with mixing blade (4 Blade Stainless Steel, 2.5" diameter). The stir rate was increased to 8000 RPM as the solution thickens, with continued mixing at 8000 rpm for an additional 300 seconds.

### 1 wt % Konjac Gum Stock Solution

494.61 grams of Water were added to a clean mixing vessel (1-liter glass beaker, VWR). 0.39 grams Koralone B-119 were added to the vessel, and stirred until homogeneously mixed. Add 5.0 grams Konjac Gum were added quickly into the vessel while mixing at 5000 RPM using the Ross Mill Mixer fitted with mixing blade (4 Blade Stainless Steel, 2.5" diameter). The stir rate was increased to 8000 RPM as the solution thickens, with continued mixing at 8000 RPM for an additional 300 seconds.

### Concentrate with Liquid Benefit Agent (Table 1)

Thoroughly cleaned mixing vessel (1-liter glass beaker, VWR) and added a magnetic stir bar. Added LBA (Cardamom Ginger Lemongrass Natural Fragrance) to the mixing vessel. 1 wt % Xanthan Gum Stock Solution was added to the vessel. 1 wt % Konjac Gum Stock Solution was added to the vessel. The vessel was placed on the magnetic stir plate and agitated at a speed sufficient to create a vortex in the mixture. The mixture was agitated until completely homogeneous.

### Dilution to Aqueous Composition (Table 2)

Water was added to a clean mixing vessel (1-liter glass beaker, VWR). The mixture was agitated with the Ross Mill Mixer fitted with mixing blade (4 Blade Stainless Steel, 2.5" diameter). The agitation speed was set to be sufficient to create a vortex in the mixture without excess foaming. The following materials were added in order into the vessel mixing, ensuring the mixture was homogeneous after each addition: Water, Ethanol, Alpha CD, Lactic Acid, Koralone B-119 and Citric Acid. After 10 minutes, Hydroxypropyl Beta CD was added to the vessel. The resulting mixture was trimmed with sodium hydroxide until reaching a pH of 7.4 (Orion, Thermo-Scientific, cat. no. 2115000). The mixture was agitated for a final 10 minutes.

### Parameter Measurements

The aqueous composition was tested in accordance with the following methods:
The sprayability was determined by the SPRAY TEST METHOD;
The viscosity of the LBA measured by VISCOSITY OF LIQUID BENEFIT AGENT TEST METHOD.

**Table 1. Example A Concentrate with Liquid Benefit Agent**

| | |
|---|---|
| Xanthan Gum Stock Solution | 7.2 g |
| Konjac Gum Stock Solution | 10.8 g |
| LBA: Cardamom Ginger Lemongrass Natural Fragrance | 0.6 g |
| LBA Concentration in Concentrate | 3.22 wt% |
| h _{LBA} | 1.000 mPa·s |

**Table 2. Example A Dilution to Aqueous Composition**

| | |
|---|---|
| Water | 546 g |
| Ethanol | 19.5 g |
| Alpha CD | 0.9 g |
| Lactic Acid | 1.4 g |
| Koralone B-119 | 0.45 g |
| Concentrate (Table 1) | 18.6 g |
| Hydroxy propyl BCD | 9.75 g |
| Sodium Hydroxide | 3.25 g |
| Final pH | 7.4 |
| LBA Concentration in Aqueous Composition | 0.10 wt% |
| Spray Grade | Sprayable |

### Example B

An inventive sample that demonstrates the preparation of a fabric treatment composition, with a very low viscosity perfume oil liquid benefit agent (Example B). The perfume oil is emulsified and stabilized in a product concentrate. Then, the product concentrate is diluted with water to form a sprayable, stable aqueous composition with acceptable mean drop size distribution.

### Materials

Ethanol (94.3%, Equistar Chemicals)
Alpha Cyclodextrin (Sigma Aldrich Product code C4642)
Diethylene Glycol (99.6%, Indorama Ventures LLC)
Citric Acid, at 50% active (Univar)
Koralone B119 (The Dow Chemical Company)
Konjac Gum (Nutricol® XP 3464, FMC Corp)
Xanthan gum (Jungbunzlauer Inc.)
Hydroxypropyl Beta Cyclodextrin (Cavasol W7 HP TL, 40%, Wacker Biosolutions)
Sodium Hydroxide (50% Membrane Grade Brenntag Mid-South, Inc) diluted to 5 %;
Cardamom Ginger Lemongrass Natural Fragrance (Procter & Gamble Company)
Water, deionized (Millipore, 18 MW)

### Preparation

### 1 wt % Xanthan Gum Stock Solution

494.61 grams of Water were added to a clean mixing vessel (1-liter glass beaker, VWR). 0.39 grams Koralone B-119 were added to the beaker, and stirred until homogeneously mixed. 5.0 grams Xanthan Gum were added quickly into the beaker while mixing at 5000 RPM using the Ross Mill Mixer fitted with mixing blade (4 Blade Stainless Steel, 2.5" diameter). The stir rate was increased to 8000 RPM as the solution thickens, with continued mixing at 8000 RPM for an additional 300 seconds.

### 1 wt % Konjac Gum Stock Solution

494.61 grams Water were added to a clean main mixing vessel (1-liter glass beaker, VWR). 0.39 grams Koralone B-119 were added to the vessel, and stirred until homogeneously mixed. Add 5.0 grams Konjac Gum were added quickly into the vessel while mixing at 5000 RPM using the Ross Mill Mixer fitted with mixing blade (4 Blade Stainless Steel, 2.5" diameter). The stir rate was increased to 8000 RPM as the solution thickens, with continued mixing at 8000 RPM for an additional 300 seconds.

### Concentrate with Liquid Benefit Agent (Table 3)

Thoroughly cleaned mixing vessel (100 ml glass beaker, VWR) and added a magnetic stir bar. Added LBA (Cardamom Ginger Lemongrass Natural Fragrance) to the mixing vessel. 1 wt % Xanthan Gum Stock Solution was added to the vessel. 1 wt % Konjac Gum Stock Solution was added to the vessel. The vessel was placed on the magnetic stir plate and agitated at a speed sufficient to create a vortex in the mixture. The mixture was agitated until completely homogeneous.

### Dilution to Aqueous Composition (Table 4)

Water was added to a clean main mixing vessel (1-liter glass beaker, VWR). The mixture was agitated with the overhead prop mixer IKA Model RW20DZM.nfitted with mixing blade (4 Blade Stainless Steel, 2.5" diameter). The agitation speed was set to create a vortex in the mixture without excess foaming. The following materials were added in order into the vessel mixing, ensuring the mixture was homogeneous after each addition: Water, Ethanol, Koralone B-119, Citric Acid. After 10 minutes of mixing, Hydroxypropyl Beta CD was added to the vessel, and mixed for an additional 600 seconds. The resulting mixtures had a pH of 6.4 (measured by Orion, Thermo-Scientific, cat. no. 2115000).

### Parameter Measurements

The Aqueous Composition was tested in accordance with the following methods:
The sprayability was determined by the SPRAY TEST METHOD;
Yield stress was determined by RHEOLOGY TEST METHOD;
Diameter of the LBA Droplets was determine by the DROP SIZE TEST METHOD;
The viscosity of the LBA measured by VISCOSITY OF LIQUID BENEFIT AGENT TEST METHOD.

**Table 3. Example B Concentrate with Liquid Benefit Agent**

| | |
|---|---|
| Xanthan Gum Stock Solution | 7.2 g |
| Konjac Gum Stock Solution | 10.8 g |
| Cardamom Ginger Lemongrass Natural Fragrance | 0.66 g |
| LBA Concentration in Concentrate | 3.53 wt% |
| h _{LBA} | 1.000 mPa·s |

**Table 4. Example B Dilution to Aqueous Composition**

| | |
|---|---|
| Water | 551.6 g |
| Ethanol | 19.5 g |
| Citric Acid | 0.18 g |
| Koralone B-119 | 0.45 g |
| Concentrate (Table 3) | 18.66 g |
| Hydroxy Propyl BCD | 9.68 g |
| Final pH | 6.4 |
| LBA Concentration in Aqueous Composition | 0.10 wt% |
| YS _{aqu.} | 56 mPa |
| Spray Grade | Sprayable |
| *D̅* _{LBA} | 30.9 µm |

### Examples C-E

Inventive samples that demonstrates the preparation of hair and surface treatment compositions, with very wide range of viscosities from 58 mPa·s (Example D) to 288 mPa·s (Example C) to 4,545 mPa·s (Example E). The LBA is emulsified and stabilized in a concentrate. The concentrate is diluted with water to form a sprayable, stable aqueous composition with acceptable mean drop size distribution.

### Materials

Konjac Gum (Nutricol® XP 3464, FMC Corp, lot 11926051)
Water (Millipore, 18 MW)
Preservative, mixture of 0.2 wt% Benzyl alcohol (Ineos Maastricht BV, Benzyl alcohol NF lot 6M07AE1), 0.2 wt% Euxyl PE 9010 (Schuelke & Mayr Gmbh, euxyl® PE 9010 preservative lot 1310481) and 0.3 wt% Symdiol 68 (Symrise, Symdiol® 68 preservative lot 10300058)
Xanthan gum (Jungbunzlauer Inc., CAS-No. 11138-66-2, lot 2532585)
PDMS (Gelest, DMS-T35, lot 4H-23386)
Argan Oil (BASF, Lipofructyl® Argan LS 9779 Argania Spinosa Kernel Oil, lot 001552044)
Liquid Benefit Agent: 0.1 wt% 10,000 mPa·s amodimethicone (Momentive, Y14945, lot 14NWFA182)
0.2 wt% cyclopentasiloxane (Sigma Aldrich, lot MKCC9214)

All samples were prepared in a 60-gram or 100-gram capacity Speed Mixer Cup (FlackTek, item code 501 222t) and mixing was done in a FlackTek DAC 150.1 FVZ-K Speed Mixer.

### Concentrate with Liquid Benefit Agent (Table 5)

Konjac Gum was added into the Speed Mixer Cup containing a pre-weighed amount of Water, Preservative and Perfume. The sample was mixed at 3500 RPM for 300 seconds. Xanthan Gum was added into the Speed Mixer Cup and was mixed at 3500 RPM for another 300 seconds. The resulting mixture was left undisturbed for 24 hours. The Liquid Benefit Agent was added to the Speed Mixer Cup. The sample was mixed again at 3500 RPM for another 300 seconds.

### Dilution to Aqueous Composition (Table 6)

An amount of the Concentrate was added to a beaker (VWR Heavy-Duty Low Form Beaker, cat #10536-390) and diluted with Water using a stand mixer (VWR VOS power control, cat #03.306886) with a propeller blade (3 blade, 1" radius) set to 500 RPM for 300 seconds, to achieve the final Aqueous Composition.

### Parameter Measurements

The Aqueous Composition was tested in accordance with the following methods:
The sprayability is determined by the SPRAY TEST METHOD;
Yield stress is determined by RHEOLOGY TEST METHOD;
Diameter of the LBA Droplets is determine by the DROP SIZE TEST METHOD;
The viscosity of the LBA measured by VISCOSITY OF LIQUID BENEFIT AGENT TEST METHOD.

**Table 5. Concentrate with Liquid Benefit Agent**

| | Example C | Example D | Example E |
|---|---|---|---|
| wt. Konjac Gum | 0.2103 g | 0.1201 g | 0.1203 g |
| wt. DI water | 92.4473 g | 95.4038 g | 95.4067 g |
| wt. Preservative | 4.9221 g | 2.8020 g | 2.818 g |
| wt. Perfume | 0.1 g | 0.1 g | 0.1 g |
| wt. Xanthan Gum | 0.1400 g | 0.0800 g | 0.0801 g |
| Wt. LBA | 2.1459 g | 1.2030 g | 1.2008 g |
| LBA | Silicone Blend | Argan Oil | T35 PDMS |
| LBA Concentration in Concentrate | 2.15 wt% | 1.21 wt% | 1.20 wt% |
| h _{LBA} | 288 mPa·s | 58 mPa·s | 4,545 mPa·s |

**Table 6. Dilute to Aqueous Composition**

| | Example C | Example D | Example E |
|---|---|---|---|
| wt. Concentrate | 8.57 g | 15.00 g | 15.02 g |
| wt. Water | 51.41 g | 45.02 g | 44.96 g |
| LBA Concentration in Aqueous Composition | 0.31 wt% | 0.30 wt% | 0.30 wt% |
| YS _{aqu.} | NM | 338 mPa | 305 mPa |
| Spray Grade | Sprayable | Sprayable | Sprayable |
| *D̅* _{LBA} | 33.6 µm | 11.5 µm | 18.0 µm |

### Examples F-G

Inventive samples that demonstrate the preparation of two hair treatment or surface treatment aqueous compositions containing blends of liquid benefit agent and solid perfume particles, by different mixing routes. For the first aqueous composition (Example F), perfume capsules are dispersed in a concentrate and liquid benefit agent (silicone blend) is emulsified and stabilized in a second concentrate. The two concentrates are mixed with water to create the final aqueous composition. For the second aqueous composition (Example G), the perfume capsule concentrate is diluted with water to create pre-aqueous composition 1 and the liquid benefit agent (silicone blend) is diluted with water to create pre-aqueous composition 2. These two pre-aqueous compositions were blended to create the final aqueous composition.

### Materials

Water (Millipore Corporations, 18 MW)
Xanthan Gum (CPK Keltrol 1000, LOT 6J3749K)
Konjac Gum (Nutricol® XP 3464, FMC, LOT 11926051)
Benzyl Alcohol (Spectrum, LOT 7F14AN1)
Euxyl PE 9010 (Schulke, LOT 7M20AN1)
SymDiol 68 (Symrise, LOT 1310481)
Perfume Capsules (Encapsys, LOT 2018-6479)
Liquid Benefit Agents: Silicone Blend with Y-14945 01P (Momentive, LOT 18FWFA462)
Cyclopentasiloxane (Momentive, LOT 17CWFA324)

All preparations were done with a speed mixer (Flacktek DAC 150.1 FVZ-K) utilizing either the Max 20 Speed Mixer Cup (Flacktek, Max 20 Translucent, 501 224t), the Max 60 Speed Mixer Cup (Flacktek, Max 60 Cup Translucent, 501 222t), or the Max 100 Speed Mixer Cup (Flacktek, Max 100 Translucent, 501 221Mt).

### Preparation of 1 wt % Xanthan Gum Stock Solution

0.106 grams Benzyl Alcohol, 0.104 grams Euxyl PE 9010, 0.158 grams SymDiol 68 at 49.156 grams of Water were added to a Max 60 Speed Mixer cup. 0.505 grams Xanthan Gum were added to the cup. The cup was placed in the speed mixer at 3500 RPM for 300 seconds.

### Preparation of 1 wt % Konjac Gum Stock Solution

0.100 grams Benzyl Alcohol, 0.105 grams Euxyl PE 9010, 0.152 grams SymDiol 68 at 49.151 grams of Water were added to a Max 60 Speed Mixer cup. 0.504 grams Konjac Gum were added to the cup. The cup was placed in the speed mixer at 3500 RPM for 300 seconds.

### Preparation of Silicone Blend Stock

6.704 grams cyclopentasiloxane and 3.302 grams Y-14945 were added to a Max 20 Speed Mixer cup. The cup was placed in the speed mixer at 3500 RPM for 300 seconds.

### Concentrate with Liquid Benefit Agent (Table 7)

### Preparation of Concentrate with Perfume Microcapsules

Benzyl alcohol, Euxyl PE 9010, SymDiol 68 and Water were added to a Max 100 Speed Mixer cup. Xanthan Gum Stock Solution and Konjac Gum Stock Solution were added to the cup and mixed at 2500 RPM for 300 seconds. Finally, 1.803 grams of Perfume Capsules were added to the cup. The cup was placed in the speed mixer at 2500 RPM for 300 seconds.

### Preparation of Concentrate with Silicone Blend

Benzyl Alcohol, Euxyl PE 9010, SymDiol 68 and Water were added to a Max 100 Speed Mixer cup. Xanthan Gum Stock Solution and Konjac Gum Stock Solution were added to the cup and mixed at 2500 RPM for 300 seconds. Finally, the Silicone Blend Stock was added to the cup. The cup was placed in the Speed Mixer at 2500 RPM for 300 seconds.

### Dilution to Aqueous Composition (Table 8)

### Preparation Aqueous Composition 1

Water, Concentrate with Perfume Microcapsules, Concentrate with Silicone Blend, Benzyl Alcohol, Euxyl PE 9010 and SymDiol 68 were added to a Max 100 Speed Mixer cup. The cup was placed in the Speed Mixer at 2500 RPM for 300 seconds.

### Preparation Aqueous Composition 1

Pre-Aqueous Composition 1 was prepared by adding Water, Concentrate with Perfume Microcapsules, Benzyl Alcohol, Euxyl PE 9010 and SymDiol 68 to Max 100 Speed Mixer cup. The cup was placed in the speed mixer at 2500 RPM for 300 seconds.

Pre-Aqueous Composition 2 was prepared by adding Water, Concentrate with Silicone Blend, Benzyl Alcohol, Euxyl PE 9010 and SymDiol 68 to Speed Mixer cup. The cup was placed in the speed mixer at 2500 RPM for 300 seconds.

### Preparation Aqueous Composition 2

Pre-Aqueous Composition 1 and Pre-Aqueous Composition 2 were added to Speed Mixer cup. The cup was placed in the speed mixer at 2500 RPM for 300 seconds.

### Parameter Measurements

The Aqueous Composition was tested in accordance with the following methods:
The sprayability is determined by the SPRAY TEST METHOD;
Yield stress is determined by RHEOLOGY TEST METHOD;
Diameter of the LBA Droplet is determine by the DROP SIZE TEST METHOD;
The viscosity of the LBA measured by VISCOSITY OF LIQUID BENEFIT AGENT TEST METHOD.

**Table 7. Concentrate with Liquid Benefit Agent**

| Concentrate with Perfume Capsules | | |
|---|---|---|
| | Example F | Example G |
| wt. Preservative - Benzyl Alcohol | 0.202 g | 0.202 g |
| wt. Preservative - Euxyl PE 9010 | 0.203 g | 0.203 g |
| wt. Preservative - SymDiol 68 | 0.308 g | 0.308 g |
| wt. Water | 67.507 g | 67.507 g |
| wt. Xanthan Gum Stock Solution | 12.003 g | 12.003 g |
| wt. Konjac Gum Stock Solution | 18.008 g | 18.008 g |
| Wt. Perfume Microcapsules | 1.803 g | 1.803 g |

| Concentrate with Silicone Blend | | |
|---|---|---|
| | Example F | Example G |
| wt. Preservative - Benzyl Alcohol | 0.203 g | 0.203 g |
| wt. Preservative - Euxyl PE 9010 | 0.203 g | 0.203 g |
| wt. Preservative - SymDiol 68 | 0.307 g | 0.307 g |
| wt. Water | 67.504 g | 67.504 g |
| wt. Xanthan Gum Stock Solution | 12.006 g | 12.006 g |
| wt. Konjac Gum Stock Solution | 18.001 g | 18.001 g |
| Wt. LBA | 1.804 g | 1.804 g |
| Silicone blend | | |
| LBA Concentration in Concentrate | 1.80 wt% | 1.80 wt% |
| YS _{conc.} | 1,024 mPa | 1,024 mPa |
| h_{10 conc.} | 321 mPa·s | 321 mPa·s |
| h _{LBA} | 288 mPa·s | 288 mPa·s |

**Table 8. Dilute to Aqueous Composition**

| Aqueous Composition 1 | | |
|---|---|---|
| wt. DI Water | 82.647 g | - |
| wt. Concentrate with Perfume Capsules | 8.338 g | - |
| wt. Concentrate with Silicone Blend | 8.337 g | - |
| wt. Preservative - Benzyl Alcohol | 0.208 g | - |
| wt. Preservative - Euxyl PE 9010 | 0.207 g | - |
| wt. Preservative - SymDiol 68 | 0.302 g | - |

| Pre-Aqueous Composition 1 | | |
|---|---|---|
| wt. DI Water | - | 82.635 g |
| wt. Concentrate with Perfume Capsules | - | 16.673 g |
| wt. Concentrate with Silicone Blend | - | - |
| wt. Preservative - Benzyl Alcohol | - | 0.204 g |
| wt. Preservative - Euxyl PE 9010 | - | 0.206 g |
| wt. Preservative - SymDiol 68 | - | 0.306 g |

| Pre-Aqueous Composition 1 | | |
|---|---|---|
| wt. DI Water | - | 82.630 g |
| wt. Concentrate with Perfume Capsules | - | - |
| wt. Concentrate with Silicone Blend | - | 16.671 g |
| wt. Preservative - Benzyl Alcohol | - | 0.203 g |
| wt. Preservative - Euxyl PE 9010 | - | 0.200 g |
| wt. Preservative - SymDiol 68 | - | 0.303 g |

| Aqueous Composition 2 | | |
|---|---|---|
| wt. Pre-Aqueous Composition 1 | - | 25.008 g |
| wt. Pre-Aqueous Composition 2 | - | 25.000 g |
| LBA Concentration in Aqueous Composition | 0.15 wt% | 0.60 wt% |
| YS _{aqu.} | 452 mPa | 1,380 mPa |
| Spray Grade | Sprayable | Sprayable |
| *D̅* _{LBA} | 25.6 µm | 36.3 µm |
| h_{10 aqu.} | 146 mPa·s | 213 mPa·s |

### Examples H-AJ

A combination of inventive and comparative examples demonstrates the importance of the preparation of the concentrate. The inventive samples have good yield stress and good drop sizes, as Example H (Peppermint Oil) and Example AC (Argan Oil). One comparative example has no yield stress, as Example N (Peppermint Oil). Other comparative examples have a yield stress but the viscosity of the concentrate preparation is insufficient to emulsify the liquid benefit agents resulting in very large drops, as with Example AH (T31 Oil) and Example M (Peppermint Oil).

### Materials

Xanthan gum (CPK Keltrol 1000, 7C5936K)
Konjac gum (Nutricol® XP 3464, FMC corp. 11926051)
Water (Millipore Corporations, 18 MW)
Acticide MBS preservative (Thor Gmbh, Acticide® MBS Biocide, lot RP-332371-1710)
Koralone (Koralone™ B-119 Preservative, The Dow Chemical Company, lot YY00G46902)
Liquid Benefit Agents:
   Peppermint Oil (MFR Ungerer Bethlehem USA, lot 50725K);
   Argan Oil (manufacturer, lot PL-A128787)
T31 Silicone Oil (Gelest Corporation)

All preparations were done with a speed mixer (Flacktek DAC 150.1 FVZ-K, Model) utilizing 60 milliliter Speed Mixer Cup (model).

### 1 wt % Xanthan Gum Stock Solution

0.08 grams Koralone and 99.0 grams Water were added to Speed Mixer Cup. 1.0 grams Xanthan Gum powder was added to the Speed Mixer Cup. The sample was mixed at 3500 RPM for 300 seconds in the speed mixer. The resulting sample was let standing for 12 hours on Shaker Table (VWR Standard 3500 Orbital Shaker, cat #89032-094) set at moderate speed, to ensure complete hydration of the Xanthan Gum.

### 1 wt % Konjac Gum Stock Solution

0.08 grams Koralone and 99.0 grams Water were added to Speed Mixer Cup. 1.0 grams Konjac Gum powder was added to the Speed Mixer Cup. The sample was mixed at 3500 RPM for 300 seconds in the speed mixer. The resulting sample was let standing for 12 hours on Shaker Table (VWR Standard 3500 Orbital Shaker, cat #89032-094) set at moderate speed, to ensure complete hydration of the Konjac Gum.

### Concentrate with Liquid Benefit Agent (Tables 9-14)

1 wt % Xanthan Gum Stock Solution and 1 wt % Konjac Gum Stock Solution was added to the Speed Mixer Cup. Water was added to the Speed Mixer Cup. Finally, the LBA (Peppermint Oil, Argan Oil or T31 Silicone Oil) was added to the Speed Mixer Cup. The mixture was mixed at 3500 RPM for 300 seconds.

### Parameter Measurements

The resulting preparations were measured with the following methods:
The viscosity of the LBA measured by VISCOSITY OF LIQUID BENEFIT AGENT TEST METHOD.
Viscosity of the Concentrate measured by RHEOLOGY METHOD;
Yield stress of the Concentrate measured by RHEOLOGY TEST METHOD;
Diameter of the LBA Droplet is determine by the DROP SIZE TEST METHOD.

**Table 9. Concentrate with Liquid Benefit Agent**

| | Example H | Example I | Example J | Example K | Example L | Example M-comparative |
|---|---|---|---|---|---|---|
| Wt. Xanthan Gum Stock | 10.149 g | 8.017 g | 6.011 g | 3.991 g | 2.017 g | 1.006 g |
| Wt. Konjac Gum Stock | 15.022 g | 12.019 g | 9.023 g | 6.211 g | 3.099 g | 1.539 g |
| Wt. Water | 25.077 g | 30.310 g | 35.080 g | 40.035 g | 45.029 g | 47.449 g |
| Wt. LBA | 0.511 g | 0.510 g | 0.516 g | 0.508 g | 0.541 g | 0.508 g |
| LBA | Peppermint Oil | Peppermint Oil | Peppermint Oil | Peppermint Oil | Peppermint Oil | Peppermint Oil |
| LBA Conc. in Conc. | 1.00 wt% | 1.00 wt% | 1.02 wt% | 1.00 wt% | 1.07 wt% | 1.00 wt% |
| h _{LBA} | 6.4 mPa·s | 6.4 mPa·s | 6.4 mPa·s | 6.4 mPa·s | 6.4 mPa·s | 6.4 mPa·s |
| h_{10 conc.} | 720 mPa·s | 447 mPa·s | 369 mPa·s | 308 mPa·s | 247 mPa·s | 90 mPa·s |
| YS _{conc.} | 3,667 mPa | 1,786 mPa | 1,087 mPa | 791 mPa | 702 mPa | 945 mPa |

**Table 10. Concentrate with Liquid Benefit Agent**

| | Example N-comparative | Example O-comparative | Example P | Example Q | Example R-comparative | Example S-comparative |
|---|---|---|---|---|---|---|
| Wt. Xanthan Gum Stock | 9.018 g | 8.043 g | 6.053 g | 3.033 g | 1.045 g | 0.508 g |
| Wt. Konjac Gum Stock | 1.150 g | 2.049 g | 4.013 g | 7.001 g | 8.995 g | 9.497 g |
| Wt. Water | 40.049 g | 40.135 g | 40.021 g | 40.018 g | 40.056 g | 40.027 g |
| Wt. LBA | 0.544 g | 0.521 g | 0.540 g | 0.509 g | 0.515 g | 0.502 g |
| LBA | Peppermint Oil | Peppermint Oil | Peppermint Oil | Peppermint Oil | Peppermint Oil | Peppermint Oil |
| LBA Conc. in Conc. | 0.90 wt% | 1.03 wt% | 1.07 wt% | 1.00 wt% | 1.02 wt% | 1.00 wt% |
| h _{LBA} | 6.4 mPa·s | 6.4 mPa·s | 6.4 mPa·s | 6.4 mPa·s | 6.4 mPa·s | 6.4 mPa·s |
| h_{10 conc.} | 114 mPa·s | 126 mPa·s | 167 mPa·s | 640 mPa·s | 294 mPa·s | 89 mPa·s |
| YS _{conc.} | 0.000 mPa | 0.000 mPa | 185.5 mPa | 14,200 mPa | 0.000 mPa | 0.000 mPa |

**Table 11. Concentrate with Liquid Benefit Agent**

| | Example T - comparative | Example U-comparative | Example V-comparative | Example W-comparative | Example X-comparative | Example Y-comparative |
|---|---|---|---|---|---|---|
| Wt. Xanthan Gum Stock | 7.016 g | 2.024 g | 0.504 g | 4.522 g | 5.001 g | 12.034 g |
| Wt. Konjac Gum Stock | 3.028 g | 8.009 g | 4.524 g | 0.527 g | 5.064 g | 3.053 g |
| Wt. Water | 40.160 g | 40.068 g | 45.382 g | 45.032 g | 40.070 g | 35.054 g |
| Wt. LBA | 0.532 g | 0.562 g | 0.543 g | 0.523 g | 0.516 g | 0.531 g |
| LBA | Peppermint Oil | Peppermint Oil | Peppermint Oil | Peppermint Oil | Peppermint Oil | Peppermint Oil |
| LBA Conc. in Conc. | 1.05 wt% | 1.11 wt% | 1.06 wt% | 1.03 wt% | 1.02 wt% | 1.05 wt% |
| h _{LBA} | 6.4 mPa· s | 6.4 mPa· s | 6.4 mPa· s | 6.4 mPa· s | 6.4 mPa· s | 6.4 mPa·s |
| h_{10 conc.} | 119 mPa·s | 1,139 mPa·s | 49 mPa·s | 44 mPa·s | 154 mPa·s | 236 mPa·s |
| YS _{conc.} | 0.000 mPa | 3,446 mPa | 0.000 mPa | 0.000 mPa | 279 mPa | 0.000 mPa |

**Table 12. Concentrate with Liquid Benefit Agent**

| | Example Z comparative | Example AA | Example AB |
|---|---|---|---|
| Wt. Xanthan Gum Stock | 9.030 g | 3.004 g | 1.556 g |
| wt. Konjac Gum Stock | 6.002 g | 12.002 g | 13.513 g |
| Wt. Water | 35.304 g | 35.074 g | 35.168 g |
| Wt. LBA | 0.513 g | 0.515 g | 0.522 g |
| LBA | Peppermint Oil | Peppermint Oil | Peppermint Oil |
| LBA Conc. in Conc. | 1.00 wt% | 1.02 wt% | 1.03 wt% |
| h _{LBA} | 6.4 mPa·s | 6.4 mPa·s | 6.4 mPa·s |
| h_{10 conc} | 229 mPa·s | 1,977 mPa·s | 789 mPa·s |
| YS _{conc} | 0.000 mPa | 5,743 mPa | 1,542 mPa |

**Table 13. Concentrate with Liquid Benefit Agent**

| | Example AC | Example AD - comparative | Example AE | Example AF - comparative | Example AG | Example AH - comparative |
|---|---|---|---|---|---|---|
| Wt. Xanthan Gum Stock | 6.056 g | 6.069 g | 4.039 g | 4.003 g | 1.017g | 1.028 g |
| Wt. Konjac Gum Stock | 9.074 g | 9.106 g | 6.030 g | 6.019 g | 1.582 g | 1.500 g |
| Wt. Water | 35.045 g | 35.061 g | 40.051 g | 40.015 g | 47.514 g | 47.550 g |
| Wt LBA | 0.524 g | 0.538 g | 0.532 g | 0.513 g | 0.509 g | 0.617 g |
| LBA | Argan Oil | T31 | Argan Oil | T31 | Argan Oil | T31 |
| LBA Conc. in Conc. | 1.03 wt% | 1.06 wt% | 1.03 wt% | 1.01 wt% | 1.00 wt% | 1.22 wt% |
| | | | | | | |
| h _{LBA} | 58.4 mPa·s | 970 mPa·s | 58.4 mPa·s | 970 mPa·s | 58.4 mPa·s | 970 mPa· s |
| h_{10 conc.} | 410 mPa·s | 370 mPa·s | 284 mPa· s | 301 mPa·s | 110 mPa s | 118 mPa·s |
| YS _{conc.} | 1,548 mPa | 1,442 mPa | 691 mPa | 670 mPa | 937 mPa | 1,072 mPa |
| *D̅* _{LBA} | | | | | | |

**Table 14. Concentrate with LiquidBenefit Agent**

| | Example AI | Example AJ |
|---|---|---|
| Wt. Xanthan Gum Stock | 3.039 g | 3.008 g |
| Wt. Konjac Gum Stock | 7.099 g | 7.036 g |
| Wt. Water | 40.093 g | 40.015 g |
| Wt. LBA | 0.517 g | 0.550 g |
| LBA | Argan Oil | T31 |
| LBA Conc. in Conc. | 1.02 wt% | 1.10 wt% |
| h _{LBA} | 58.4 mPa·s | 970 mPa·s |
| h_{10 conc.} | 770 mPa·s | 961 mPa·s |
| YS _{conc.} | 6,939 mPa | 4,989 mPa |
| *D̅* _{LBA} | | |

### Examples AK-AL

Inventive examples that demonstrate the preparation of a product concentrate composition, utilizing both tara gum and heat, sometimes suggested to enhance gum binding. First, the peppermint oil was emulsified into a concentrate composition containing a blend of xanthan gum and tara gum maintained at room temperature (Example AK); second, the peppermint oil was emulsified into a concentrate composition containing a blend of xanthan gum and tara gum maintained at 60 deg. C temperature (Example AL).

### Materials

Water (Millipore Corporations, 18 MW)
Xanthan Gum (CPK Keltrol 1000, lot 7C5936K)
Tara Gum (Ingredion TIC Pretested Tara Gum 100, Lot 33809)
Preservative (Koralone B19, Dow Chemical Company, Lot YY00G41902)
Liquid Benefit Agent: Peppermint Oil (MFR Ungerer Bethlehem USA, Lot 50725 K)
All preparations were done with a Speed Mixer (Flacktek DAC 150.1 FVZ-K).

### Preparation of 1 wt % Xanthan Gum Stock Solution

Add 0.047 grams of Preservatives and 49.462 grams of deionized Water to Speed Mixer Max 60 Cup (Flacktek, Max 60 Cup Translucent, 501 222t). Add 0.503 grams of Xanthan Gum powder to cup, and place cup in Speed Mixer at 3500 RPM for 300 seconds.

### Preparation of 1 wt % Tara Gum Stock Solution

Add 0.049 grams of Preservatives and 49.467 grams of deionized Water to Speed Mixer Max 60 Cup (Flacktek, Max 60 Cup Translucent, 501 222t). Add 0.502 grams of Tara Gum powder to cup, and place cup in Speed Mixer at 3500 RPM for 300 seconds.

### Concentrate with Liquid Benefit Agent (Table 15)

### Preparation of Concentrate at Room Temperature

Take Speed Mixer Max 60 Cup, add mass of Preservatives, add mass of Water, add mass of 1 wt% Xanthan Gum Stock, add mass of 1 wt% Tara Gum Stock, and add indicated mass of LBA. The Speed Mixer Max 60 Cup and all contents were mixed at indicated rate for indicated time. The composition was maintained at room temperature for 12 hours.

### Preparation of Concentrate at 60 degree Celsius

Take Speed Mixer Max 60 Cup, add mass of Preservatives, add mass of deionized Water, add mass of 1 wt% Xanthan Gum Stock, add mass of 1 wt% Tara Gum Stock, and add indicated mass of LBA. The Speed Mixer Max 60 Cup and all contents were mixed at indicated rate for indicated time. The composition was maintained at 60 °C temperature for 1 hour.

### Parameter Measurements

The resulting preparations were measured with the following methods:
Viscosity of the LBA measured by VISCOSITY OF LIQUID BENEFIT AGENT TEST METHOD;
Viscosity of the Concentrate measured by RHEOLOGY METHOD;
Yield stress of the Concentrate measured by RHEOLOGY TEST METHOD;
Diameter of the LBA Droplet is determine by the DROP SIZE TEST METHOD.

**Table 15. Concentrate with Liquid Benefit Agent**

| | Example AK | Example AL |
|---|---|---|
| Preservative | 0.044 g | 0.047 g |
| Water | 36.712 g | 36.714 g |
| Xanthan Gum Stock | 5.005 g | 5.005 g |
| Tara Gum Stock | 7.504 g | 7.500 g |
| Wt. LBA | 0.754 g | 0.751 g |
| LBA | Peppermint Oil | Peppermint Oil |
| LBA Conc. in Conc. | 1.51 wt% | 1.50 wt% |
| Temperature | 25 °C | 60 °C |
| h _{LBA} | 6.4 mPa·s | 6.4 mPa·s |
| h_{10 conc.} | 913 mPa·s | 1,608 mPa·s |
| YS _{conc.} | 4,268 mPa | 4,217 mPa |
| *D̅* _{LBA} | 25.6 µm | 36.3 µm |

### Examples AM- AR

Inventive and comparative examples demonstrate the importance of comparing the viscosity of the liquid benefit agent to the concentrate composition, in creating good drops. Examples AM - AQ have a decreasing concentration and corresponding viscosity in the concentrate composition. The drop size increases with a decreasing viscosity until Example AQ can no longer make acceptable drops, as indicated by the top 5 % of distribution. However, replacing the high viscosity liquid benefit agent (Example AR) allows the formation of acceptable drops.

### Materials

Xanthan Gum (xanthan gum, Novaxan, Dispersible, lot 140521)
Konjac Gum (konjac gum, FMC, Nutricol XP 3464, lot 11926051)
Water (Millipore Corporations, 18 MW)
Flash AS (Momentive Corporation, XX-8766, lot PE12152016)
A15 (Gelest Corporation)
Preservative (Thor Gmbh, Acticide® MBS Biocide, lot RP-332371-1710)

### Concentrate with Liquid Benefit Agent

200 grams of Water and 0.6 g of Acticide were added to a 100 ml beaker. Xanthan Gum and Konjac Gum were slowly added with overhead stirring until achieving a visually homogenous Concentrate Composition. These preparations were additionally shaken for at least another 48 more hours to ensure complete hydration of the gums. A fraction of this composition was placed into a 100-ml Speed Mixer cup (FlackTek, item code 501 222t). The Liquid Benefit Agent was added to the cup. The cup was tightly covered and placed into a Speed Mixer (FlackTek DAC 150.1 FVZ-K Speed Mixer) and mixed at 3500 RPM for 300 seconds.

### Dilute to Aqueous Composition

The resulting *aqueous mixtures* were diluted to 0.05 wt % total gum concentrations, with Water.

### Parameter Measurements

The resulting preparations were measured with the following methods:
Viscosity of the LBA was measured by VISCOSITY OF THE LIQUID BENEFIT AGENT;
Viscosity of the concentrate was measured by RHEOLOGY TEST METHOD;
Drop size diameter of the LBA in Aqueous Composition was determine by the DROP SIZE TEST METHOD;
The yield stress of the Aqueous Composition was measured by RHEOLOGY TEST METHOD.

**Table 16. Concentrate with LiquidBenefit Agent**

| | Example AM | Example AN | Example AO | Example AP -comparative | Example AQ - comparative | Example AR |
|---|---|---|---|---|---|---|
| Water | 200 g | 200 g | 200 g | 200 g | 200 g | 200 g |
| Xanthan Gum Stock | 0.505 g | 0.265 g | 0.200 g | 0.162 g | 0.081 g | 0.162 g |
| Konjac Gum Stock | 0.499 g | 0.402 g | 0.302 g | 0.241 g | 0.125 g | 0.241 g |
| Wt. Conc. | 30.681 g | 29.944 g | 29.967 g | 30.118 g | 30.036 g | 30.210 g |
| Wt. LBA | 1.030 g | 1.015 g | 1.047 g | 1.124 g | 1.040 g | 0.421 g |
| LBA | Flash | Flash | Flash | Flash | Flash | T15 |
| LBA Conc. in Conc. | 3.24 wt% | 3.27 wt% | 3.38 wt% | 3.59 wt% | 3.34 wt% | 1.37 wt% |

**Table 17. Dilution to Aqueous Composition**

| | Example AM | Example AN | Example AO | Example AP - comparative | Example AQ-comparative | Example AR |
|---|---|---|---|---|---|---|
| Wt. Conc. | 31.711 g | 30.959 g | 31.004 g | 31.242 g | 31.076 g | 30.630 g |
| Water | 90.0 g | 90.0 g | 90.0 g | 90.0 g | 90.0 g | 90.0 g |
| LBA Conc. in Aqu. | 0.85 wt% | 0.84 wt% | 0.87 wt% | 0.93 wt% | 0.80 wt% | 0.35 wt% |
| h _{LBA} | 2,450 mPa·s | 2,450 mPa·s | 2,450 mPa·s | 2,450 mPa·s | 2,450 mPa·s | 421 mPa·s |
| h_{10 conc} | 586 mPa·s | 784 mPa·s | 448 mPa·s | 201 mPa·s | 43 mPa·s | 201 mPa·s |
| YS _{conc} | 1,529 mPa | 2,748 mPa | 598 mPa | NM | NM | NM |
| *D̅* _{LBA} | 69.2 µm | 85.0 µm | 184.9 µm | NM | NM | 95.5 µm |

### Examples AS-AV

Inventive examples that demonstrate the preparation of aqueous compositions by diluting the concentrate with water, and with water combined with other ingredients in the aqueous composition. The first concentrate was prepared by emulsifying and stabilizing peppermint oil in a concentrate containing 30:70 mixture of xanthan gum to konjac gum; the second concentrate was prepared by emulsifying and stabilizing peppermint oil in a concentrate containing 60:40 mixture of xanthan gum to konjac gum. These concentrates were diluted with water (Example AS, Example U), xanthan gum stock solution (Example AT) and/or konjac gum stock solution (Example AV), to prepare the final aqueous composition.

### Materials

Water (Millipore Corporations, 18 MW)
Xanthan Gum (CPK Keltrol 1000, LOT 6J3749K)
Konjac Gum (Nutricol® XP 3464, FMC corp., LOT 11926051)
Benzyl Alcohol (Spectrum, LOT 7F14AN1)
Euxyl PE 9010 (Schulke, LOT 7M20AN1)
SymDiol 68 (Symrise, LOT 1310481)
Peppermint Oil (MFR Ungerer Bethlehem USA, Lot 50725 K)

All preparations were done with a Speed Mixer (Flacktek DAC 150.1 FVZ-K).

### Preparation of 1 wt % Xanthan gum Stock Solution

Added 0.102 grams Benzyl Alcohol, 0.104 grams Euxyl PE 9010, 0.151 grams SymDiol 68, and 49.156 grams deionized Water to Speed Mixer Max 60 Cup (Flacktek, Max 60 Cup Translucent, 501 222t). Added 0.502 grams of Xanthan Gum powder to cup, and place cup in Speed Mixer at 3500 RPM for 300 seconds.

### Preparation of 1 wt % Konjac Gum Stock Solutions

Added 0.106 grams Benzyl Alcohol, 0.108 grams Euxyl PE 9010, 0.154 grams SymDiol 68, and 49.157 grams deionized Water to Speed Mixer Max 60 Cup (Flacktek, Max 60 Cup Translucent, 501 222t). Added 0.501 grams of Konjac Gum powder to cup, and place cup in Speed Mixer at 3500 RPM for 300 seconds.

### Concentrate with Liquid Benefit Agent (Table 18)

### Preparation of 30: 70 Concentrate

Took Speed Mixer Max 60 Cup, added 0.104 grams Benzyl Alcohol, 0.105 grams Euxyl PE 9010, 0.152 grams SymDiol 68, and 33.608 grams deionized Water to Speed Mixer Max 60 Cup (Flacktek, Max 60 Cup Translucent, 501 222t). Added 4.506 grams of Xanthan Gum Stock, added 10.508 grams of Konjac Gum Stock, and placed cup in Speed Mixer at 2500 RPM for 300 seconds. Added 1.057 grams of Peppermint Oil to cup and placed cup in Speed Mixer at 2500 RPM for another 300 seconds.

### Preparation of 60: 40 Concentrate

Took Speed Mixer Max 60 Cup, added 0.102 grams Benzyl Alcohol, 0.107 grams Euxyl PE 9010, 0.152 grams SymDiol 68, and 33.308 grams deionized Water to Speed Mixer Max 60 Cup (Flacktek, Max 60 Cup Translucent, 501 222t). Added 9.002 grams of Xanthan Gum Stock, added 6.006 grams of Konjac Gum Stock, and placed cup in Speed Mixer at 2500 RPM for 300 seconds. Added 1.355 grams of Peppermint Oil to cup and placed cup in Speed Mixer at 2500 RPM for another 300 seconds.

### Dilute to Aqueous Composition (Table 19)

### Preparation of Aqueous Composition, Option 1

Took Speed Mixer Max 60 Cup and added deionized Water, 30:70 Concentrate, Benzyl Alcohol, Euxyl PE 9010 and SymDiol 68 and processed at 2500 RPM for 300 seconds. In a second example, took Speed Mixer Max 60 Cup and added deionized Water, 30:70 Concentrate, Benzyl Alcohol, Euxyl PE 9010, SymDiol 68 and Xanthan Gum Stock, and processed at 2500 RPM for 300 seconds.

### Preparation of Aqueous Composition, Option 2

In one example, took Speed Mixer Max 60 Cup and added deionized Water, 60:40 Concentrate, Benzyl Alcohol, Euxyl PE 9010 and SymDiol 68 and processed at 2500 RPM for 300 seconds. In a second example, took Speed Mixer Max 60 Cup and added deionized Water, 60:40 Concentrate, Benzyl Alcohol, Euxyl PE 9010, SymDiol 68 and Konjac Gum Stock, and processed at 2500 RPM for 300 seconds.

### Parameter Measurements:

Viscosity of the LBA was measured by VISCOSITY OF THE LIQUID BENEFIT AGENT;
Viscosity of the Aqueous Composition and Concentrate Composition was measured by RHEOLOGY TEST METHOD;
Drop size diameter of the LBA in Aqueous Composition was determine by the DROP SIZE TEST METHOD;
The yield stress of the Aqueous Composition and Concentrate Composition was measured by RHEOLOGY TEST METHOD.

**Table 18. Concentrate with Liquid Benefit Agent**

| Concentrate with 30:70 Gum Ratio | | | | |
|---|---|---|---|---|
| | Example AS | Example AT | Example AU | Example AV |
| wt. preservative - Benzyl Alcohol | 0.104 g | 0.104 g | - | - |
| wt. preservative - Euxyl PE 9010 | 0.105 g | 0.105 g | - | - |
| wt. preservative - SymDiol 68 | 0.152 g | 0.152 g | - | - |
| wt. Water | 33.308 g | 33.308 g | - | - |
| wt. Xanthan Gum Stock Solution | 4.506 g | 4.506 g | - | - |
| wt. Konjac Gum Stock Solution | 10.508 g | 10.508 g | - | - |
| Wt. LBA | 1.057 g | 1.057 g | - | - |
| LBA | Peppermint Oil | Peppermint Oil | - | - |
| LBA Conc. in Conc. | 2.11 wt% | 2.11 wt% | - | - |

| Concentrate with 60:40 Gum Ratio | | | | |
|---|---|---|---|---|
| | Example AS | Example AT | Example AU | Example AV |
| wt. preservative - Benzyl Alcohol | - | - | 0.102 g | 0.102 g |
| wt. preservative - Euxyl PE 9010 | - | - | 0.107 g | 0.107 g |
| wt. preservative - SymDiol 68 | - | - | 0.152 g | 0.152 g |
| wt. Water | - | - | 33.308 g | 33.308 g |
| wt. Xanthan Gum Stock Solution | - | - | 9.002g | 9.002 g |
| wt. Konjac Gum Stock Solution | - | - | 6.006 g | 6.006 g |
| Wt. LBA | - | - | 1.335 g | 1.335 g |
| LBA | - | - | Peppermint Oil | Peppermint Oil |
| LBA Concentration in Concentrate | - | - | 2.71 wt% | 2.71 wt% |
| YS _{conc.} | 2,854 mPa | 2,854 mPa | 219 mPa | 219 mPa |
| h_{10 conc.} | 473 mPa·s | 473 mPa·s | 196 mPa·s | 196 mPa·s |
| h _{LBA} | 6.4 mPa·s | 6.4 mPa·s | 6.4 mPa·s | 6.4 mPa·s |

**Table 19. Dilute to Aqueous Composition**

| | Example AS | Example AT | Example AU | Example AV |
|---|---|---|---|---|
| wt. Water | 82.638 g | 84.316 g | 82.636 g | 86.525 g |
| wt. Concentrate with 30:70 Gum Ratio | 16.673 g | 14.282 g | - | - |
| wt. Concentrate with 60:40 Gum Ratio | - | - | 16.678 g | 11.115 g |
| wt. preservative - Benzyl Alcohol | 0.208 g | 0.207 g | 0.206 g | 0.204 g |
| wt. preservative - Euxyl PE 9010 | 0.207 g | 0.204 g | 0.205 g | 0.206 g |
| wt. preservative - SymDiol 68 | 0.301 g | 0.308 g | 0.301 g | 0.305 g |
| wt. Xanthan Gum Stock | - | 0.715 g | - | - |
| wt. Konjac Gum Stock | - | - | - | 1.670 g |
| LBA concentration in Aqueous Composition | 0.35 wt% | 0.30 wt% | 0.45 wt% | 0.30 wt% |
| YS _{aqu.} | 903 mPa | 1,420 mPa | 21 mPa | 1,491 mPa |
| Spray Grade | Sprayable | Sprayable | Sprayable | Sprayable |
| *D̅* _{LBA} | 32.4 µm | 37.3 µm | 36.0 µm | 30.1 µm |

### Examples AW- BL

Inventive and comparative examples demonstration the important parameters in the preparation of a product concentrate. These include inventive samples, that have yield stress and good drop sizes, as Example AW - BE. They include comparative examples that have no yield stress, as Example BG - BL, resulting in different amounts and ratios of the Xanthan and Konjac Gum. Most importantly, these examples demonstrate that it is possible to emulsify up to 20 wt% Liquid Benefit Agent (Example AZ), but not effectively emulsify at 60 wt% Liquid Benefit Agent (Example BF) despite the relative viscosity and presence of a yield stress.

### Materials

Konjac gum (konjac gum, FMC, Nutricol XP 3464, lot 11926051)
Water, deionized (Millipore, 18 MW)
Preservative Acticide MBS (Thor Gmbh, Acticide® MBS biocide lot RP-332371-1710)
Xanthan gum (Xanthan gum, Jungbunzlauer, FG, lot 2532585)
0.050 Pa·s PDMS (Gelest, DMS-T15, lot 8F-13015)
5 Pa·s PDMS (Gelest, DMS-T35, lot 4H-23386)
All samples were prepared in a 60-gram or 100-gram capacity Speed Mixer Cup (FlackTek, item code 501 222t) and mixing was done in a FlackTek DAC 150.1 FVZ-K Speed Mixer.

### Concentrate with Liquid Benefit Agent

Konj ac gum was added to the Speed Mixer Cup containing Water, Preservative and Perfume. The sample was mixed at 3500 rpm for 5 minutes. Xanthan gum was weighed, and carefully and slowly added into the Speed Mixer Cup and mixed again at 3500 rpm for another 5 minutes. If any noticeable undissolved gum chunks are noticed, the sample is further mixed 3500 rpm for another 5 minutes until the gums are completely dissolved. The resulting mixture was left undisturbed for 24 hours. The *liquid benefit agent* was added to the Speed Mixer Cup. The sample was mixed again at 3500 rpm for another 5 minutes.

### Dilute to Aqueous Composition

Some of the *Concentrate* was removed, added to a plastic jar and diluted with Water. The mixture was agitated with the Ross mill mixer fitted with mixing blade (4 Blade Stainless Steel, 2.5" diameter) (or equivalent) to 500 rpm for 300 seconds, to achieve the Aqueous Composition.

### Parameter Measurements

The resulting preparations were measured with the following methods:
Viscosity of the LBA was measured by VISCOSITY OF LIQUID BENEFIT AGENT TEST METHOD
Viscosity of the concentrate was measured by RHEOLOGY TEST METHOD
Drop size diameter of the LBA in Aqueous Composition was determine by the DROP SIZE TEST METHOD
The yield stress of the Aqueous Composition was measured by RHEOLOGY TEST METHOD

**Table 20. Concentrate with Liquid Benefit Agent**

| | Example AW | Example AX | Example AY |
|---|---|---|---|
| wt. Konjac Gum | 0.0726 g | 0.1201 g | 0.1203 g |
| wt. Water | 59.5027 g | 58.7172 g | 56.1877 |
| wt. Preservative | 0.0362 g | 0.0366 g | 0.0362 g |
| wt. Xanthan Gum | 0.0726 g | 0.0753 g | 0.0724 g |
| wt. LBA | 0.3618 g | 1.0810 g | 3.6081 g |
| LBA | T15 | T15 | T15 |
| LBA Conc. in Conc. | 0.60 wt% | 1.80 wt% | 6.00 wt% |
| YS _{conc.} | 1,295 mPa | 2,263 mPa | 3,786 mPa |
| h_{10 Conc.} | 429 mPa·s | 586 mPa·s | 945 mPa·s |
| h _{LBA} | 48.2 mPa·s | 48.2 mPa·s | 48.2 mPa·s |

**Table 21: Dilution to Aqueous Composition**

| | Example AW | Example AX | Example AY |
|---|---|---|---|
| wt. Gum Concentrate | 10.00 g | 10 .00g | 10.00 g |
| wt. Water | 49.96 g | 49.99 g | 50.00 g |
| LBA concentration in Aqueous Composition | 0.10 wt% | 0.30 wt% | 1.00 wt% |
| YS _{aqu.} | 45 mPa | 91 mPa | 44 mPa |
| *D̅* _{LBA} | 24 µm | 30 µm | 203 µm |

**Table 22. Concentrate with LiquidBenefit Agent**

| | Example AZ | Example BA | Example BB |
|---|---|---|---|
| wt. Konjac Gum | 0.1087 g | 0.1082 g | 0.1093 g |
| wt. Water | 48.9846 g | 59.4717 g | 58.7077 g |
| wt. Preservative | 0.0363 g | 0.0360 g | 0.0361 g |
| wt. Xanthan Gum | 0.0726 g | 0.0727 g | 0.0723 g |
| wt. LBA | 10.8140 g | 0.3617 g | 1.0802 g |
| LBA | T15 | T15 | T15 |
| LBA Conc. in Conc. | 18.01 wt% | 0.60 wt% | 1.80 wt% |
| YS _{conc.} | 7,795 mPa | 5,994 mPa | 8,959 mPa |
| h_{10 Conc.} | 2,214 mPa·s | 1,489 mPa·s | 2,565 mPa·s |
| h _{LBA} | 48.2 mPa· s | 48.2 mPa·s | 48.2 mPa· s |

**Table 23: Dilution to Aqueous Composition**

| | Example AZ | Example BA | Example BB |
|---|---|---|---|
| wt. Gum Concentrate | 10.00 g | 10 .00g | 10.00 g |
| wt. Water | 50.09 g | 50.07 g | 50.02 g |
| LBA concentration in Aqueous Composition | 3.00 wt% | 0.10 wt% | 0.30 wt% |
| YS _{aqu.} | 218 mPa | 101 mPa | 39 mPa |
| *D̅* _{LBA} | 184 µm | 21.7 µm | 82.8 µm |

**Table 24. Concentrate with LiquidBenefit Agent**

| | Example BC | Example BD | Example BE |
|---|---|---|---|
| wt. Konjac Gum | 0.1090 g | 0.1086 g | 0.1442 g |
| wt. Water | 56.1864 g | 48.9854 g | 58.7045 g |
| wt. Preservative | 0.0362 g | 0.0372 g | 0.0370 g |
| wt. Xanthan Gum | 0.0732 g | 0.0721 g | 0.0361 g |
| wt. LBA | 3.6009 g | 10.8085 g | 1.0830 g |
| LBA | T15 | T15 | T15 |
| LBA Conc. in Conc. | 6.00 wt% | 18.01 wt% | 1.80 wt% |
| YS _{conc.} | 9,601 mPa | 11,835 mPa | 7,662 mPa |
| h_{10 Conc.} | 2,499 mPa·s | 2,768 mPa· | 1,928 mPa |
| h _{LBA} | 48.2 mPa·s | 48.2 mPa·s | 48.2 mPa·s |

**Table 25: Dilution to Aqueous Composition**

| | Example BC | Example BD | Example BE |
|---|---|---|---|
| wt. Gum Concentrate | 10.00 g | 10 .00g | 10.00 g |
| wt. Water | 50.07 g | 50.04 g | 50.01 g |
| LBA concentration in Aqueous Composition | 1.00 wt% | 3.00 wt% | 0.30 wt% |
| YS _{aqu.} | 143 mPa | 44 mPa | NM |
| *D̅* _{LBA} | 58.6 µm | NM | 120 µm |

**Table 26. Concentrate with LiquidBenefit Agent**

| | Example BF - comparative | Example BG - comparative | Example BH - comparative |
|---|---|---|---|
| wt. Konjac Gum | 0.1096 g | 0.1813 g | 0.1622 g |
| wt. Water | 23.7919 g | 58.7040 g | 58.7091 g |
| wt. Preservative | 0.0362 | 0.0369 | 0.0365 |
| wt. Xanthan Gum | 0.0735 g | 0.0000 g | 0.0187 g |
| wt. LBA | 35.9702 g | 1.0822 g | 1.0806 g |
| LBA | T15 | T35 | T35 |
| LBA Conc. in Conc. | 60.00 wt% | 1.80 wt% | 1.80 wt% |
| YS _{conc.} | 14,443 mPa | 0 mPa | 0 mPa |
| h_{10 Conc.} | 3,407 mPa·s | 65 mPa·s | 821 mPa |
| h _{LBA} | 48.2 mPa·s | 4,545 mPa·s | 4,545 mPa·s |

**Table 27: Dilution to Aqueous Composition**

| | Example BF - comparative | Example BG - comparative | Example BH - comparative |
|---|---|---|---|
| wt. Gum Concentrate | 10 g | 10 g | 10.01 g |
| wt. Water | 50.07 g | 49.96 g | 49.99 g |
| LBA concentration in Aqueous Composition | 10.00 wt% | 0.30 wt% | 0.30 wt% |
| YS _{aqu.} | 18 mPa | 0 mPa | 0 mPa |
| *D̅* _{LBA} | > 1000 µm | > 1000 µm | > 1000 µm |

**Table 28. Concentrate with LiquidBenefit Agent**

| | Example BI (comp) | Example BJ (comp) | Example BK (comp) |
|---|---|---|---|
| wt. Konjac Gum | 0.0722 g | 0.0367 g | 0.0181 g |
| wt. Water | 58.7113 g | 58.7133 g | 58.7050 g |
| wt. Preservative | 0.0362 | 0.0363 | 0.0368 |
| wt. Xanthan Gum | 0.1088 g | 0.1441 g | 0.1622 g |
| wt. LBA | 1.0799 g | 1.0819 g | 1.0802 g |
| LBA | T35 | T35 | T35 |
| LBA Conc. in Conc. | 1.80 wt% | 1.80 wt% | 1.80 wt% |
| YS _{conc.} | 841 mPa | 0 mPa | 0 mPa |
| h_{10 Conc.} | 363 mPa·s | 283 mPa·s | 256 mPa·s |
| h _{LBA} | 4,545 mPa·s | 4,545 mPa·s | 4,545 mPa·s |

**Table 29: Dilution to Aqueous Composition**

| | Example BI (comp) | Example BJ (comp) | Example BK (comp) |
|---|---|---|---|
| wt. Gum Concentrate | 10.00 g | 10.02 g | 10.02 g |
| wt. Water | 50.04 g | 50.02 g | 50.03 g |
| LBA concentration in Aqueous Composition | 0.30 wt% | 0.30 wt% | 0.30 wt% |
| YS _{aqu.} | 0 mPa | 0 mPa | 0 mPa |
| *D̅* _{LBA} | > 1000 µm | > 1000 µm | > 1000 µm |

**Table 30. Concentrate with LiquidBenefit Agent**

| | Example BL-comparative |
|---|---|
| wt. Konjac Gum | 0.0000 g |
| wt. Water | 58.7157 g |
| wt. Preservative | 0.0369 |
| wt. Xanthan Gum | 0.1801 g |
| wt. LBA | 1.0816 g |
| LBA | T35 |
| LBA Conc. in Conc. | 1.80 wt% |
| YS _{conc.} | 0 mPa |
| h_{10 Conc.} | 170 mPa·s |
| h _{LBA} | 4,545 mPa·s |

**Table 31: Dilution to Aqueous Composition**

| | Example BL-comparative |
|---|---|
| wt. Gum Concentrate | 10.02 g |
| wt. Water | 50.04 g |
| LBA concentration in Aqueous Composition | 0.30 wt% |
| YS _{aqu.} | 0 mPa |
| *D̅* _{LBA} | > 1000 µm |

### Examples BM-BP

Inventive examples that demonstrate the preparation of aqueous compositions from a single concentrate each containing on benefit agent (Example BM and Example BN), from a blend of two concentrates with the benefit agents (example BO) and from a single concentrate prepared with both benefit agents (BP).

### Materials

Xanthan gum (Xanthan gum, CPK Keltrol 1000, 7C5936K)
Konjac gum (Konjac gum (Nutricol® XP 3464, FMC corp. 11926051)
Water (Millipore Corporations, 18 MW)
Silicone Oil, Y14945 (Momentive, Y14945, lot 14NWFA182)
Silicone Oil, Cyclopentasiloxane (Sigma Aldrich, 444278 Decamethylcyclopentasiloxane, lot MKCC9214)
Perfume capsules (Encapsys, LOT 2018-6479)
Benzyl Alcohol (Spectrum, LOT 7F14AN1)
Euxyl PE 9010 (Schulke, LOT 7M20AN1)
SymDiol 68 (Symrise, LOT 1310481)

All preparations were done with a Speed Mixer (Flacktek DAC 150.1 FVZ-K, Model)

### 1 wt % Xanthan Gum Stock Solution

0.106 grams Benzyl Alcohol, 0.106 grams Euxyl Alcohol, 0.151 grams SymDiol 68 were added to 49.154 grams Water in a 60 ml Speed Mixer cup (part). 0.505 grams of Xanthan Gum were carefully added to the cup. The mixture was processed in the Speed Mixer at 3500 RPM for 300 seconds.

### 1 wt % Konjac Gum Stock Solution

0.101 grams Benzyl Alcohol, 0.100 grams Euxyl Alcohol, 0.157 grams SymDiol 68 were added to 49.153 grams Water in a 60 ml Speed Mixer cup (part). 0.502 grams of konjac gum were carefully added to the cup. The mixture was processed in the Speed Mixer at 3500 RPM for 300 seconds.

### Silicone Blend Stock Solution

Blend 3.308 grams Y14945 silicone oil and 6.708 grams cyclopentasiloxane silicone oil. The mixture was processed in the Speed Mixer at 3500 RPM for 300 seconds.

### Gum Concentrate with Liquid Benefit Agent

### Concentrates with Benefit Agent

### Concentrate with Perfume Capsules

Water, Preservative, Xanthan gum Stock Solution and Konjac gum Stock Solution were added to 50 ml Speed Mixer Cup, with perfume capsules. The mixture was mixed at 2500rpm for 300 seconds.

### Concentrate with Silicone Blend

Water, Preservative, Xanthan gum Stock Solution and Konjac gum Stock Solution were added to 50 ml Speed Mixer Cup, with silicone blend. The mixture was mixed at 2500rpm for 300 seconds.

### Concentrate with Perfume Capsules and Silicone Blend

Water, Preservative, Xanthan gum Stock Solution and Konjac gum Stock Solution were added to 60 mL Speed Mixer Cup, with perfume capsules and silicone blend. The mixture was mixed at 2500rpm for 300 seconds.

### Dilute to Aqueous Compositions

### Preparation of Complete

Added Water to 100 ml Speed Mixer Cup. Then, added the proper amounts of Benzyl Alcohol, Euxyl PE 9010 and
SymDiol 68. Finally, the different concentrates are added. The mixture was mixed at 2500 rpm for 300 seconds.

**Table 32. Concentrate with LiquidBenefit Agent**

| Concentrate (A) with Perfume Capsules | | | | |
|---|---|---|---|---|
| | BM | BN | BO | BP |
| wt. DI water | 31.241 g | - | 31.241 g | - |
| wt. Xanthan gum Stock | 6.004 g | - | 6.004 g | - |
| wt. Konjac Stock | 9.006 g | - | 9.006 g | - |
| wt. Benzyl Alcohol | 0.103 g | - | 0.103 g | - |
| wt. Euxyl PE 9010 | 0.101 g | - | 0.101 g | - |
| wt. SymDiol 68 | 0.156 g | - | 0.156 g | - |
| wt. Perfume Capsules | 3.412 g | - | 3.412 g | - |

| Concentrate (B) with Silicone Blend | | | | |
|---|---|---|---|---|
| | BM | BN | BO | BP |
| wt. DI water | - | 33.755 g | 33.755 g | - |
| wt. Xanthan gum Stock | - | 6.006 g | 6.006 g | - |
| wt. Konjac Stock | - | 9.009 g | 9.009 g | - |
| wt. Benzyl Alcohol | - | 0.108 g | 0.108 g | - |
| wt. Euxyl PE 9010 | - | 0.105 g | 0.105 g | - |
| wt. SymDiol 68 | - | 0.157 g | 0.157 g | - |
| wt. Silicone Blend | - | 0.905 g | 0.905 g | - |

| Concentrate (C) with Perfume Capsules and Silicone Blend | | | | |
|---|---|---|---|---|
| | BM | BN | BO | BP |
| wt. DI water | - | - | - | 33.458 g |
| wt. Xanthan gum Stock | - | - | - | 6.002 g |
| wt. Konjac Stock | - | - | - | 9.001 g |
| wt. Benzyl Alcohol | - | - | - | 0.105 g |
| wt. Euxyl PE 9010 | - | - | - | 0.108 g |
| wt. SymDiol 68 | - | - | - | 0.154 g |
| wt. Perfume Capsules and Silicone Blend | - | - | - | 0.905 g PC + 0.307 Silicone Blend |

**Table 33: Dilution to Aqueous Composition**

| | BM | BN | BO | BP |
|---|---|---|---|---|
| wt. DI Water | 82.633 g | 82.638 g | 82.642 g | 82.638 g |
| wt. Concentrate (A) | 16.676 g | 0.000 g | 8.338 g | 0.000 g |
| wt. Concentrate (B) | 0.000 g | 16.672 g | 8.330 g | 0.000 g |
| wt. Concentrate (C) | 0.000 g | 0.000 g | 0.000 g | 16.679 g |
| wt. Benzyl Alcohol | 0.208 g | 0.205 g | 0.203 g | 0.206 g |
| wt. Euxyl PE 9010 | 0.208 g | 0.203 g | 0.207 g | 0.206 g |
| wt. SymDiol 68 | 0.305 g | 0.304 g | 0.302 g | 0.303 g |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

It should be understood that every maximum numerical limitation given throughout this specification will include every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A sprayable product comprising:
a) a spray dispenser; and
b) an aqueous composition disposed in the spray dispenser, the aqueous composition comprising an aqueous phase and liquid benefit agent droplets homogeneously and discontinuously dispersed throughout the aqueous phase, wherein the aqueous phase comprises a structurant system comprising a first polysaccharide and a second polysaccharide that is different from the first polysaccharide, wherein the aqueous phase comprises less than 10,000 ppm surfactant, and wherein the aqueous composition exhibits a yield stress as determined by the RHEOLOGY TEST METHOD.

2. The sprayable product according to Claim 1, wherein the first polysaccharide comprises xanthan gum and the second polysaccharide is selected from the group consisting of glucomannan, galactomannan, or combinations thereof.

3. The sprayable product according to Claim 2, where the second polysaccharide is selected from the group consisting of: konjac gum, tara gum, locust bean gum, or combination thereof.

4. The sprayable product according to any of the preceding claims, wherein the structurant system is present at a level of less than or equal to 0.5 wt.%, based on the total weight of the aqueous composition.

5. The sprayable product according to any of the preceding claims, wherein the first polysaccharide is present at a level of greater than 10 wt. % and less than 90 wt. %, based on the total weight of the structurant system.

6. The sprayable product according to any of the preceding claims, wherein the aqueous phase comprises less than 100 ppm surfactant, preferably the aqueous composition does not comprise an effective amount of a surfactant.

7. The sprayable product according to any of the preceding claims, where the aqueous composition comprises greater than 90 wt. % water.

8. The sprayable product according to any of the preceding claims, wherein the liquid benefit agent is selected from the group consisting of silicone, aminosilicone, D5, PDMS, essential oil, natural oil, perfume oil, polyoil, and combinations thereof.

9. The sprayable product according to any of the preceding claims, wherein the liquid benefit agent droplets have a diameter of less than 300 µm determined by the DROPLET SIZE TEST METHOD.

10. The sprayable product according to any of the preceding claims, wherein the liquid benefit agent droplets comprise less than 20 wt. % of the aqueous composition.

11. An aqueous composition comprising an aqueous phase and liquid benefit agent droplets discontinuously dispersed throughout the aqueous phase, wherein the aqueous composition is made by a process comprising the steps of:
a) mixing a first polysaccharide and a second polysaccharide in water to form a concentrated aqueous phase, wherein the first polysaccharide is different from the second polysaccharide;
b) emulsifying a liquid benefit agent into the concentrated aqueous phase to form liquid benefit agent droplets discontinuously dispersed through the concentrated aqueous phase; and
c) diluting the concentrated aqueous phase additional water to form the aqueous composition, wherein the concentration of the structurant system in the concentrated aqueous phase is greater than the concentration of the structurant system in the aqueous composition.

12. The aqueous composition according to claim 11 further comprising a plurality of solid particles.

13. The aqueous composition according to claim 11 or Claim 12, wherein the liquid benefit agent droplets comprise two or more different liquid benefit agents.

14. A method of formulating an aqueous composition, the method comprising the steps of:
a) dispersing a structurant system into water to form a concentrate, the structurant system comprising a first polysaccharide and a second polysaccharide that is different from the first polysaccharide;
b) mixing a liquid benefit agent into the concentrate to form liquid benefit agent droplets dispersed throughout the concentrate; and
c) diluting the concentrate with additional water to form the aqueous composition, wherein the concentration of the structurant system in the concentrate is greater than the concentration of the structurant system in the aqueous composition.

15. The method according to claim 14, wherein the concentrate is a first concentrate, wherein the liquid benefit agent is a first liquid benefit agent, the method further comprising the steps of:
a) dispersing a structurant system into water to form a second concentrate, the structurant system comprising a first polysaccharide and a second polysaccharide that is different from the first polysaccharide;
b) mixing a second liquid benefit agent into the second concentrate to form second liquid benefit agent droplets discontinuously dispersed throughout the concentrated aqueous phase, wherein the first and second liquid benefit agents have different viscosities;
c) mixing the first and second concentrates; and
d) diluting the first and second concentrates with additional water, wherein the concentration of the structurant system in the second concentrate is greater than the concentration of the structurant system in the aqueous composition.
